# EUROPEAN PATENT APPLICATION

(11) **EP 4 144 729 A1**
(43) Date of publication of application: **08.03.2023**
(21) Application number: 21823056.3
(22) Date of filing: 09.06.2021
(51) Int. Cl.: C07D 409/14, C07D 413/14, G02C 7/00, C08K 5/45, C08L 101/00, G02B 1/04

(54) **COMPOSITION AND COMPOUND**

(30) Priority: 10.06.2020 JP 2020101102
(71) Applicant: FUJIFILM Corporation, Tokyo 106-8620 (JP)
(72) Inventor: AMASAKI, Ichiro, Fujinomiya-shi, Shizuoka 418-8666 (JP); KIMURA, Keizo, Fujinomiya-shi, Shizuoka 418-8666 (JP); SASAKI, Daisuke, Fujinomiya-shi, Shizuoka 418-8666 (JP); TANI, Yukio, Fujinomiya-shi, Shizuoka 418-8666 (JP)
(74) Representative: Hoffmann Eitle
(86) International application number: PCT/JP2021/021938
(87) International publication number: WO 2021/251433

(57) **Abstract**

The present disclosure provides a composition containing a compound represented by General Formula (1) and a polymer compound, in which a content of a chloride ion is less than 1.5 ppm with respect to a total mass of the composition, and provides a compound.

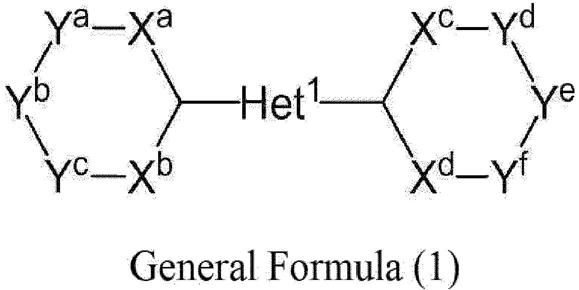

In General Formula (1), Het¹ represents a divalent aromatic heterocyclic residue of a 5-membered ring or a 6-membered ring, X^{a}, X^{b}, X^{c}, and X^{d} each independently represent a heteroatom, Y^{a}, Y^{b}, Y^{c}, Y^{d}, Y^{e}, and Y^{f} each independently represent a heteroatom or a carbon atom, and two 6-membered rings bonded to Het¹ each independently may have a double bond.

## Description

### BACKGROUND OF THE INVENTION

### 1. Field of the Invention

The present disclosure relates to a composition and a compound.

### 2. Description of the Related Art

A benzoxazinone-based compound is known as one kind of compound capable of absorbing ultraviolet rays or blue light (see, for example, JP5236297B and JP2018/180929A). Such a compound as described above can be used in combination with, for example, a polymer compound to impart the absorbability of ultraviolet rays or blue light to various articles. An article containing a compound capable of absorbing ultraviolet rays or blue light and a polymer compound can be used as, for example, a lens and a film.

### SUMMARY OF THE INVENTION

Among the quality standards required for an article including a compound capable of absorbing ultraviolet rays or blue light and a polymer compound, the color standard has been stricter than in the related art, for example, in a field such as optics. In particular, the color standard has required reducing redness.

An object of an aspect of the present disclosure is to provide a composition having less redness.

An object of another aspect according to the present disclosure is to provide a compound that suppresses an increase in redness in the presence of a polymer compound.

The present disclosure includes the following aspects.
<1> A composition containing a compound represented by General Formula (1) and a polymer compound, in which a content of a chloride ion is less than 1.5 ppm with respect to a total mass of the composition.

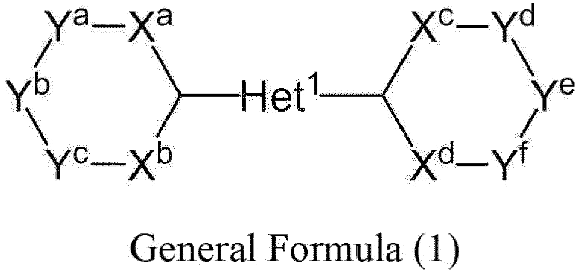

In General Formula (1), Het¹ represents a divalent aromatic heterocyclic residue of a 5-membered ring or a 6-membered ring, X^{a}, X^{b}, X^{c}, and X^{d} each independently represent a heteroatom, Y^{a}, Y^{b}, Y^{c}, Y^{d}, Y^{e}, and Y^{f} each independently represent a heteroatom or a carbon atom, and two 6-membered rings bonded to Het¹ each independently may have a double bond.

<2> The composition according to <1>, in which a content of a sodium ion is less than 1.5 ppm with respect to the total mass of the composition.

<3> The composition according to <1> or <2>, in which the compound represented by General Formula (1) is a compound represented by General Formula (2).

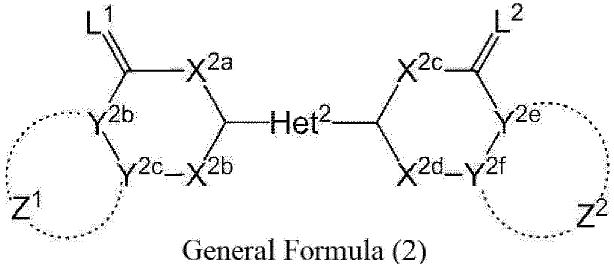

In General Formula (2), Het² represents a divalent aromatic heterocyclic residue of a 5-membered ring or a 6-membered ring, X^{2a}, X^{2b}, X^{2c}, and X^{2d} each independently represent a heteroatom, Y^{2b}, Y^{2c}, Y^{2e}, and Y^{2f} each independently represent a heteroatom or a carbon atom, two 6-membered rings bonded to Het² may each independently have a double bond, L¹ and L² each independently represent an oxygen atom, a sulfur atom, or NR^{a}, where R^{a} represents a hydrogen atom or a monovalent substituent, Z¹ represents an atomic group that is required for bonding to Y^{2b} and Y^{2c} to form a 4-membered ring to an 8-membered ring, and Z² represents an atomic group that is required for bonding to Y^{2e} and Y^{2f} to form a 4-membered ring to an 8-membered ring.

<4> The composition according to <3>, in which the compound represented by General Formula (2) is a compound represented by General Formula (3).

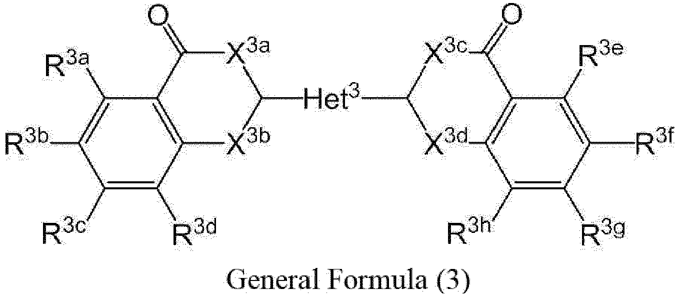

In General Formula (3), Het³ represents a divalent aromatic heterocyclic residue of a 5-membered ring or a 6-membered ring, X^{3a}, X^{3b}, X^{3c}, and X^{3d} each independently represent a heteroatom, X^{3a} or X^{3b} may form a double bond with a carbon atom in a ring bonded to Het³, X^{3c} or X^{3d} may form a double bond with a carbon atom in a ring bonded to Het³, and R^{3a}, R^{3b}, R^{3c}, R^{3d}, R^{3e}, R^{3f}, R^{3g}, and R^{3h} each independently represent a hydrogen atom or a monovalent substituent.

<5> The composition according to <4>, in which the compound represented by General Formula (3) is a compound represented by General Formula (4).

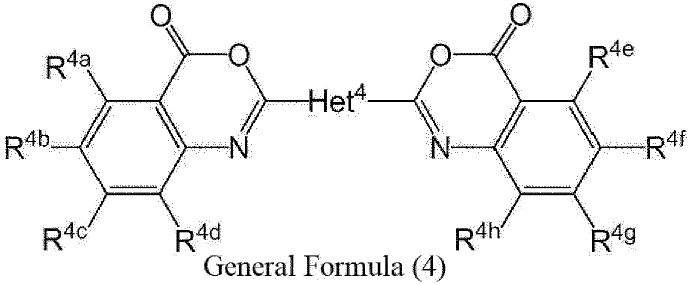

In General Formula (4), Het⁴ represents a divalent aromatic heterocyclic residue of a 5-membered ring or a 6-membered ring, and R^{4a}, R^{4b}, R^{4c}, R^{4d}, R^{4e}, R^{4f}, R^{4g}, and R^{4h} each independently represent a hydrogen atom or a monovalent substituent.

<6> The composition according to <5>, in which the compound represented by General Formula (4) is a compound represented by General Formula (5).

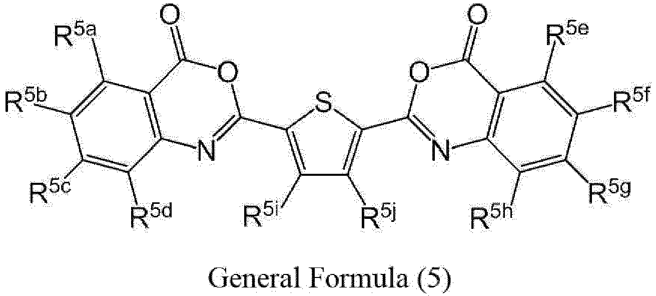

In General Formula (5), R^{5a}, R^{5b}, R^{5c}, R^{5d}, R^{5e}, R^{5f}, R^{5g}, and R^{5h} each independently represent a hydrogen atom or a monovalent substituent, and R⁵ⁱ and R^{5j} each independently represent a hydrogen atom or a monovalent substituent.

<7> The composition according to any one of <1> to <6>, in which the polymer compound is at least one selected from the group consisting of polyester, polycarbonate, polyethylene, polypropylene, an acrylic resin, cyclic polyolefin, an epoxy resin, a polyurethane, polythiourethane, polyimide, polyamide, and a fluororesin.

<8> The composition according to any one of <1> to <7>, in which the composition is for a lens for spectacles.

<9> The composition according to any one of <1> to <7>, in which the composition is for an optical lens.

<10> The composition according to any one of c<1> to <7>, in which the composition is for an optical film.

<11> A compound represented by General Formula (1), General Formula (2), General Formula (3), General Formula (4), or General Formula (5), in which a content of a chloride ion is less than 30 ppm in terms of mass.

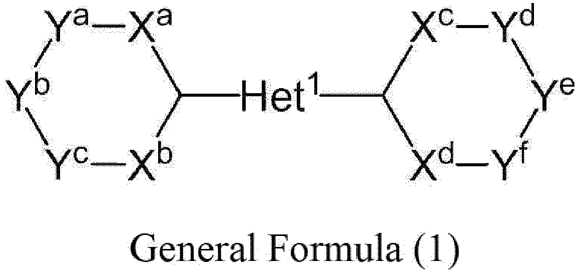

In General Formula (1), Het¹ represents a divalent aromatic heterocyclic residue of a 5-membered ring or a 6-membered ring, X^{a}, X^{b}, X^{c}, and X^{d} each independently represent a heteroatom, Y^{a}, Y^{b}, Y^{c}, Y^{d}, Y^{e}, and Y^{f} each independently represent a heteroatom or a carbon atom, and two 6-membered rings bonded to Het¹ each independently may have a double bond.

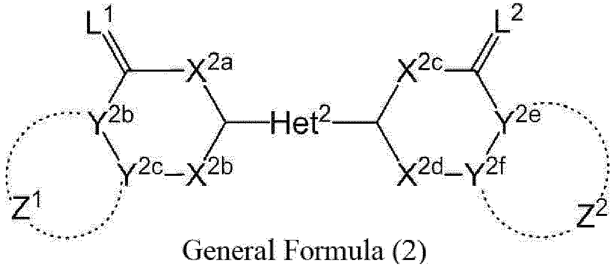

In General Formula (2), Het² represents a divalent aromatic heterocyclic residue of a 5-membered ring or a 6-membered ring, X^{2a}, X^{2b}, X^{2c}, and X^{2d} each independently represent a heteroatom, Y^{2b}, Y^{2c}, Y^{2e}, and Y^{2f} each independently represent a heteroatom or a carbon atom, two 6-membered rings bonded to Het² may each independently have a double bond, L¹ and L² each independently represent an oxygen atom, a sulfur atom, or NR^{a}, where R^{a} represents a hydrogen atom or a monovalent substituent, Z¹ represents an atomic group that is required for bonding to Y^{2b} and Y^{2c} to form a 4-membered ring to an 8-membered ring, and Z² represents an atomic group that is required for bonding to Y^{2e} and Y^{2f} to form a 4-membered ring to an 8-membered ring.

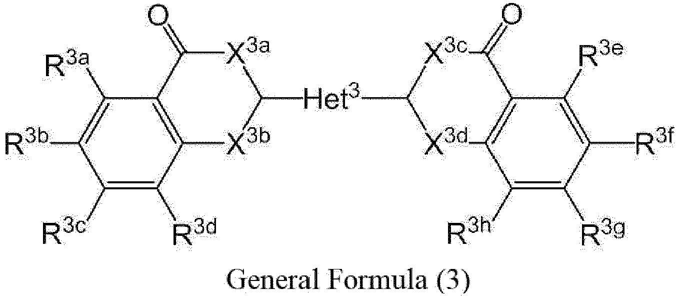

In General Formula (3), Het³ represents a divalent aromatic heterocyclic residue of a 5-membered ring or a 6-membered ring, X^{3a}, X^{3b}, X^{3c}, and X^{3d} each independently represent a heteroatom, X^{3a} or X^{3b} may form a double bond with a carbon atom in a ring bonded to Het³, X^{3c} or X^{3d} may form a double bond with a carbon atom in a ring bonded to Het³, and R^{3a}, R^{3b}, R^{3c}, R^{3d}, R^{3e}, R^{3f}, R^{3g}, and R^{3h} each independently represent a hydrogen atom or a monovalent substituent.

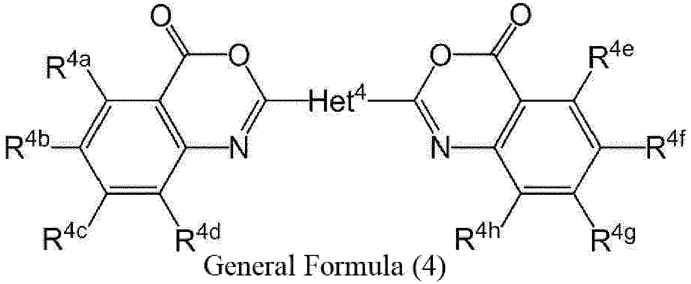

In General Formula (4), Het⁴ represents a divalent aromatic heterocyclic residue of a 5-membered ring or a 6-membered ring, and R^{4a}, R^{4b}, R^{4c}, R^{4d}, R^{4e}, R^{4f}, R^{4g}, and R^{4h} each independently represent a hydrogen atom or a monovalent substituent.

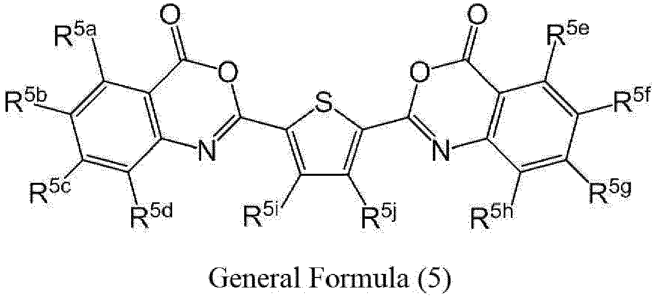

In General Formula (5), R^{5a}, R^{5b}, R^{5c}, R^{5d}, R^{5e}, R^{5f}, R^{5g}, and R^{5h} each independently represent a hydrogen atom or a monovalent substituent, and R⁵ⁱ and R^{5j} each independently represent a hydrogen atom or a monovalent substituent.

<12> The compound according to <11>, in which a content of a sodium ion is less than 1 ppm in terms of mass, a content of an aluminum ion is less than 0.5 ppm in terms of mass, a content of an iron ion is less than 0.5 ppm in terms of mass, and a content of a calcium ion is less than 1 ppm in terms of mass.

According to the aspect of the present disclosure, a composition having less redness is provided.

According to another aspect according to the present disclosure, there is provided a compound that suppresses an increase in redness in the presence of a polymer compound.

### DESCRIPTION OF THE PREFERRED EMBODIMENTS

Hereinafter, embodiments according to the present disclosure will be described in detail. The present disclosure is not limited in any way to the following embodiments and may be implemented with appropriate modifications within the scope of the purpose of the present disclosure.

The numerical range indicated by using "to" in the present disclosure indicates a range including numerical values described before and after "to" as a lower limit value and an upper limit value, respectively. Regarding numerical ranges that are described stepwise in the present disclosure, an upper limit value or a lower limit value described in a numerical value may be replaced with an upper limit value or a lower limit value of another stepwise numerical range. In addition, regarding a numerical range described in the present disclosure, an upper limit value or a lower limit value described in a numerical value may be replaced with a value described in Examples.

In the present disclosure, the amount of each component in a composition means, in a case where the composition contains a plurality of substances corresponding to such a component, the total amount of the plurality of substances in the composition, unless otherwise specified.

In the present disclosure, the term "step" includes not only an independent step but also a step that cannot be clearly distinguished from other steps, as long as the intended purpose of the step is achieved.

In the present disclosure, "% by mass" and "% by weight" are synonymous, and "parts by mass" and "parts by weight" are synonymous.

In the present disclosure, a combination of two or more preferred aspects is a more preferred aspect.

In the present disclosure, "room temperature" means 25°C unless otherwise specified.

### <Compound>

A compound according to one embodiment of the present disclosure is represented by General Formula (1), in which the content of chloride ions is less than 30 ppm in terms of mass. According to the above embodiment, there is provided a compound that suppresses an increase in redness in the presence of a polymer compound.

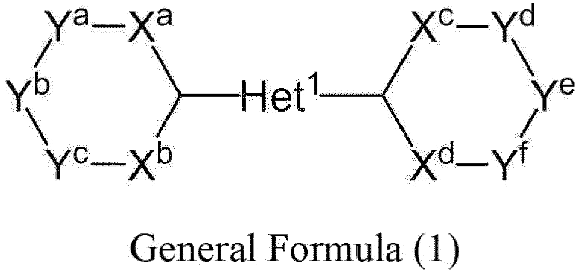

In General Formula (1), Het¹ represents a divalent aromatic heterocyclic residue of a 5-membered ring or a 6-membered ring, X^{a}, X^{b}, X^{c}, and X^{d} each independently represent a heteroatom, Y^{a}, Y^{b}, Y^{c}, Y^{d}, Y^{e}, and Y^{f} each independently represent a heteroatom or a carbon atom, and two 6-membered rings bonded to Het¹ each independently may have a double bond.

The reason why the compound according to one embodiment of the present disclosure exhibits the above-described effect is presumed as follows. As will be described later, the compound according to one embodiment of the present disclosure can be used as a component of various articles together with a polymer compound. The above article can be manufactured, for example, through a process of mixing a compound represented by General Formula (1) with a polymer compound. However, for example, ions in the manufacturing process of the above article, particularly chloride ions, may cause hydrolysis of the polymer compound. In addition, it is conceived that the compound represented by General Formula (1) is hydrolyzed due to the influence of the chloride ion and the influence of a hydrolyzate of the polymer compound, which results in an increase in redness. It is conceived that most of the chloride ions present in the manufacturing step are derived from the compound represented by General Formula (1). On the other hand, according to the compound according to one embodiment of the present disclosure, since such hydrolysis as described above can be suppressed by setting the content of chloride ions in the compound represented by General Formula (1) to less than 30 ppm, an increase in redness can be suppressed.

### [Ion]

### (Chloride ion)

in terms of mass, the content of chloride ions in the compound represented by General Formula (1) is less than 30 ppm, preferably less than 25 ppm, more preferably less than 20 ppm, and particularly preferably less than 10 ppm. In a case where the content of chloride ions is less than 30 ppm, it is possible to suppress an increase in redness in the presence of the polymer compound. In addition, in a case of reducing the content of chloride ions in the compound represented by General Formula (1), it is possible to suppress the generation of a substance having a low affinity (for example, compatibility) with respect to the polymer compound, thereby suppressing an increase in haze. It is conceived that the substance having a low affinity with respect to the polymer compound is generated, for example, by further condensation of a hydrolyzate of the compound represented by General Formula (1), which has been generated due to the influence of the chloride ions. The lower limit of the content of chloride ions is not limited. For example, the content of chloride ions in the compound represented by General Formula (1) may be 0 ppm or more in terms of mass. The content of chloride ions is measured by combustion ion chromatography. As the measuring device, a combustion device "AQF-100" manufactured by Mitsubishi Chemical Corporation, and an ion chromatography "ICS-1500" manufactured by Dionex Corporation are used.

### (Ion other than chloride ion)

It is preferable to reduce not only the content of chloride ions but also the content of ions other than chloride ions. In a case of reducing the content of ions other than the chloride ions in addition to the chloride ions, it is possible to further suppress an increase in redness in the presence of the polymer compound. In addition, in a case of reducing the content of ions other than the chloride ions in addition to the chloride ions, it is possible to further suppress an increase in haze in the presence of the polymer compound. Examples of ions other than chloride ions include metal ions. Examples of the metal ion include a sodium ion, an aluminum ion, an iron ion, and a calcium ion. Hereinafter, a preferred range of the content of ions other than chloride ions will be described.

### - Sodium ion -

In a certain embodiment, the content of sodium ions in the compound represented by General Formula (1) is preferably less than 10 ppm, more preferably less than 5 ppm, still more preferably less than 1 ppm, and particularly preferably less than 0.5 ppm, in terms of mass. In a case where the content of sodium ions is in the above range, it is possible to suppress an increase in redness in the presence of the polymer compound. In addition, it is possible to suppress an increase in haze in the presence of the polymer compound. The lower limit of the content of sodium ions is not limited. For example, the content of sodium ions in the compound represented by General Formula (1) may be 0 ppm or more in terms of mass. The content of sodium ions is measured according to a frameless atomic absorption method. A polarized Zeeman atomic absorption spectrophotometer "ZA3700" manufactured by Hitachi, Ltd. is used as the measuring device.

### - Aluminum ion -

In a certain embodiment, the content of aluminum ions in the compound represented by General Formula (1) is preferably less than 1 ppm, more preferably less than 0.5 ppm, and particularly preferably less than 0.3 ppm, in terms of mass. In a case where the content of aluminum ions is in the above range, it is possible to further suppress an increase in redness in the presence of the polymer compound. In addition, it is possible to suppress an increase in haze in the presence of the polymer compound. The lower limit of the content of aluminum ions is not limited. For example, the content of aluminum ions in the compound represented by General Formula (1) may be 0 ppm or more in terms of mass. The content of aluminum ions is measured according to a method equivalent to the above-described method of measuring the content of sodium ions.

### - Iron ion -

In a certain embodiment, the content of iron ions in the compound represented by General Formula (1) is preferably less than 1 ppm and more preferably less than 0.5 ppm in terms of mass. In a case where the content of iron ions is in the above range, it is possible to further suppress an increase in redness in the presence of the polymer compound. In addition, it is possible to suppress an increase in haze in the presence of the polymer compound. The lower limit of the content of iron ions is not limited. For example, the content of iron ions in the compound represented by General Formula (1) may be 0 ppm or more in terms of mass. The content of iron ions is measured according to a method equivalent to the above-described method of measuring the content of sodium ions.

### - Calcium ion -

In a certain embodiment, the content of calcium ions in the compound represented by General Formula (1) is preferably less than 1 ppm, more preferably less than 0.8 ppm, and particularly preferably less than 0.6 ppm, in terms of mass. In a case where the content of calcium ions is in the above range, it is possible to further suppress an increase in redness in the presence of the polymer compound. In addition, it is possible to suppress an increase in haze in the presence of the polymer compound. The lower limit of the content of calcium ions is not limited. For example, the content of calcium ions in the compound represented by General Formula (1) may be 0 ppm or more in terms of mass. The content of calcium ions is measured according to a method equivalent to the above-described method of measuring the content of sodium ions.

Hereinafter, a preferred relationship between the "chloride ion" and the "ion other than the chloride ion" will be described. In a certain embodiment, it is preferable to reduce the content of sodium ions in addition to the chloride ions. In a certain embodiment, it is more preferable to reduce the content of sodium ions, aluminum ions, iron ions, and calcium ions in addition to the chloride ions. For example, in a certain embodiment, it is preferable that the content of sodium ions is less than 1 ppm in terms of mass, the content of aluminum ions is less than 0.5 ppm in terms of mass, the content of iron ions is less than 0.5 ppm in terms of mass, and the content of calcium ions is less than 1 ppm in terms of mass.

From the viewpoint of suppressing an increase in redness in the presence of the polymer compound and suppressing an increase in haze in the presence of the polymer compound, it is preferable to reduce the total content of "chloride ions" and "ions other than the chloride ions". In a certain embodiment, the total content of chloride ions and sodium ions is preferably less than 20 ppm, more preferably less than 15 ppm, and particularly preferably less than 10 ppm, in terms of mass. In a certain embodiment, the total content of chloride ions, sodium ions, aluminum ions, iron ions, and calcium ions is preferably less than 40 ppm, more preferably less than 20 ppm, and particularly preferably less than 15 ppm, in terms of mass. The lower limit of the total content of the plurality of ions described above is not limited. The total content of the plurality of ions described above may be determined in a range of 0 ppm or more in terms of mass.

Examples of the method of reducing the content of each of the above-described ions include a method of washing the compound represented by General Formula (1) with water. The washing method will be described in the section of "Production method" described below. In addition, the content of each of the above-described ions can also be reduced by producing the compound represented by General Formula (1) using a raw material having high purity.

### [Chemical structure]

The compound according to one embodiment of the present disclosure has a chemical structure represented by General Formula (1).

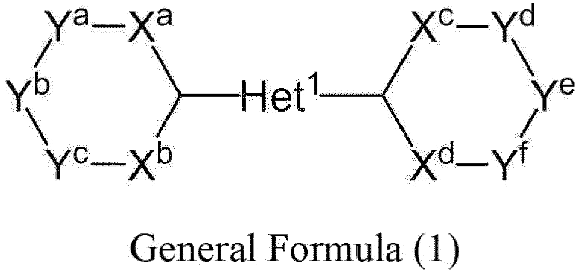

In General Formula (1), Het¹ represents a divalent aromatic heterocyclic residue of a 5-membered ring or a 6-membered ring. The divalent aromatic heterocyclic residue is formally a divalent group obtained by removing any two atoms or atomic groups bonded to the aromatic heterocyclic ring of the aromatic heterocyclic compound. In other words, the basic skeleton of the aromatic heterocyclic residue represented by Het¹ is a 5-membered or 6-membered aromatic heterocyclic ring. Hereinafter, the aromatic heterocyclic residue represented by Het¹ will be specifically described.

The aromatic heterocyclic residue contains a heteroatom in the ring. Examples of the heteroatom include a boron atom, a nitrogen atom, an oxygen atom, a silicon atom, a phosphorus atom, a sulfur atom, a selenium atom, and a tellurium atom. From the viewpoints of the durability of the compound and the availability of the raw material, the aromatic heterocyclic residue preferably contains a nitrogen atom, an oxygen atom, or a sulfur atom, more preferably contains an oxygen atom or a sulfur atom, and particularly preferably contains a sulfur atom, in the ring. The number of heteroatoms contained in the aromatic heterocyclic residue may be one or two or more. In a case where the aromatic heterocyclic residue contains a plurality of heteroatoms, the plurality of heteroatoms may include the same kind of heteroatoms or two or more kinds of heteroatoms.

The aromatic heterocyclic residue includes a 5-membered or 6-membered aromatic heterocyclic ring. The aromatic heterocyclic ring may be a 5-membered or 6-membered monocyclic ring or a fused ring including a 5-membered or 6-membered ring. The aromatic heterocyclic residue preferably has a monocyclic structure. Examples of the aromatic heterocyclic ring include a pyrrole ring, a pyrazole ring, an imidazole ring, a 1,2,3-triazole ring, a 1,2,4-triazole ring, a pyridine ring, a pyridazine ring, a pyrimidine ring, a pyrazine ring, a 1,3,5-triazine ring, a furan ring, a thiophene ring, an oxazole ring, an isoxazole ring, a thiazole ring, an isothiazole ring, a 1,2,3-oxadiazole ring, and a 1,3,4-thiadiazole ring. From the viewpoints of the durability of the compound and the availability of the raw material, as the aromatic heterocyclic ring, the aromatic heterocyclic residue preferably includes a pyrrole ring, a pyridine ring, a furan ring, or a thiophene ring, more preferably includes a furan ring or a thiophene ring, and particularly preferably include a thiophene ring.

The aromatic heterocyclic ring which is the basic skeleton of the aromatic heterocyclic residue may have a substituent. Examples of the substituent that is capable of being introduced into the aromatic heterocyclic ring include a monovalent substituent. Examples of the monovalent substituent include a halogen atom (for example, a fluorine atom, a chlorine atom, a bromine atom, or an iodine atom), an alkyl group having 1 to 20 carbon atoms (for example, a methyl group or an ethyl group), an aryl group having a number of 6 to 20 carbon atoms (for example, a phenyl group or a naphthyl group), a cyano group, a carboxy group, an alkoxycarbonyl group (for example, a methoxycarbonyl group), an aryloxycarbonyl group (for example, a phenoxycarbonyl group), a substituted or unsubstituted carbamoyl group (for example, a carbamoyl group, an N-phenylcarbamoyl group, or an N,N-dimethylcarbamoyl group), an alkylcarbonyl group (for example, an acetyl group), an arylcarbonyl group (for example, a benzoyl group), a nitro group, a substituted or unsubstituted amino group (for example, an amino group, a dimethylamino group, or anilino group), an acylamino group (for example, an acetoamide group or an ethoxycarbonylamino group), a sulfonamide group (for example, a methanesulfonamide group), an imide group (for example, a succinimide or a phthalimide group), an imino group (for example, a benzylideneamino group), a hydroxy group, an alkoxy group having 1 to 20 carbon atoms (for example, a methoxy group), an aryloxy group (for example, a phenoxy group), an acyloxy group (for example, an acetoxy group), an alkylsulfonyloxy group (for example, a methanesulfonyloxy group), an arylsulfonyloxy group (for example, a benzenesulfonyloxy group), a sulfo group, a substituted or unsubstituted sulfamoyl group (for example, a sulfamoyl group or an N-phenylsulfamoyl), an alkylthio group (for example, a methylthio group), an arylthio group (for example, a phenylthio group), an alkylsulfonyl group (for example, a methanesulfonyl group), an arylsulfonyl group (for example, a benzenesulfonyl group), and a heterocyclic group having 6 to 20 carbon atoms (for example, a pyridyl group or a morpholino group). The number of substituents on the aromatic heterocyclic ring may be one or two or more. In a case where the number of substituents on the aromatic heterocyclic ring is plural, the plurality of substituents may include the same kind of substituents or two or more kinds of substituents. The plurality of substituents may be bonded to each other to form a ring. In a case where a substituent is introduced into the aromatic heterocyclic ring, the substituent is preferably at least one selected from the group consisting of an alkyl group, an alkoxy group, and an aryl group, more preferably at least one selected from the group consisting of an alkyl group and an aryl group, and particularly preferably an alkyl group. The substituent on the aromatic heterocyclic ring may further have a substituent. Examples of the substituent that is capable of being introduced into the substituent on the aromatic heterocyclic ring include the above-described monovalent substituent.

Next, two 6-membered rings bonded to Het¹ in General Formula (1) will be specifically described. The compound represented by General Formula (1) contains a 6-membered ring composed of X^{a}, X^{b}, Y^{a}, Y^{b}, Y^{c}, and a carbon atom, and a 6-membered ring composed of X^{c}, X^{d}, Y^{d}, Y^{e}, Y^{f}, and a carbon atom. The chemical structure of the two 6-membered rings bonded to Het¹ is not limited as long as the above-described constitutional element is contained in the ring. The two 6-membered rings bonded to Het¹ may have the same chemical structure or chemical structures different from each other. It is preferable that the two 6-membered rings bonded to Het¹ have the same chemical structure.

In General Formula (1), X^{a}, X^{b}, X^{c}, and X^{d} each independently represent a heteroatom. Examples of the heteroatom include a boron atom, a nitrogen atom, an oxygen atom, a silicon atom, a phosphorus atom, a sulfur atom, a selenium atom, and a tellurium atom. X^{a}, X^{b}, X^{c}, and X^{d} are each independently preferably a nitrogen atom, an oxygen atom, or a sulfur atom, and preferably a nitrogen atom or an oxygen atom. For example, the nitrogen atom, which is one kind of heteroatom, may form a double bond with one of two adjacent atoms in the ring and form a single bond with the other atom. The heteroatom represented by X^{a}, X^{b}, X^{c}, and X^{d} may be bonded to a hydrogen atom or a substituent. For example, the nitrogen atom, which is one kind of heteroatom, may be bonded to two adjacent atoms in the ring and be bonded to a hydrogen atom or a monovalent substituent. Examples of the substituent that is capable of being introduced into the heteroatom include a monovalent substituent that is capable of being introduced into the above-described aromatic heterocyclic ring.

In General Formula (1), Y^{a}, Y^{b}, Y^{c}, Y^{d}, Y^{e}, and Y^{f} each independently represent a heteroatom or a carbon atom. Examples of the heteroatom include a nitrogen atom, an oxygen atom, and a sulfur atom. It is preferable that Y^{a}, Y^{b}, Y^{c}, Y^{d}, Y^{e}, and Y^{f} are each independently a carbon atom. The heteroatom represented by Y^{a}, Y^{b}, Y^{c}, Y^{d}, Y^{e}, and Y^{f} may be bonded to a hydrogen atom or a substituent. Examples of the substituent that is capable of being introduced into the heteroatom include a monovalent substituent that is capable of being introduced into the above-described aromatic heterocyclic ring. The carbon atom represented by Y^{a}, Y^{b}, Y^{c}, Y^{d}, Y^{e}, and Y^{f} may be bonded to a hydrogen atom or a substituent. Examples of the substituent that is capable of being introduced into the carbon atom include a monovalent substituent that is capable of being introduced into the above-described aromatic heterocyclic ring. The carbon atom represented by Y^{a}, Y^{b}, Y^{c}, Y^{d}, Y^{e}, and Y^{f} may form a group containing a carbon atom in the ring. Examples of the group containing a carbon atom include =CH-, -CH₂-, and a carbonyl group (-C(=O)-). At least two of Y^{a}, Y^{b}, and Y^{c} may further form a ring (for example, a 4-membered ring to an 8-membered ring). At least two of Y^{d}, Y^{e}, and Y^{f} may further form a ring (for example, a 4-membered ring to an 8-membered ring).

In General Formula (1), the two 6-membered rings bonded to Het¹ may each independently have a double bond. As described above, for example, the nitrogen atom, which is one kind of the heteroatoms represented by X^{a}, X^{b}, X^{c}, and X^{d}, can form "=N-" in the ring. As described above, for example, the carbon atom represented by Y^{a}, Y^{b}, Y^{c}, Y^{d}, Y^{e}, and Y^{f} can form "=CH-" in the ring.

In General Formula (1), the two 6-membered rings bonded to Het¹ may each independently form a fused ring. As described above, for example, at least two of Y^{a}, Y^{b}, and Y^{c} further form a ring, or at least two of Y^{d}, Y^{e}, and Y^{f} further form a ring, whereby a fused ring can be formed. It is preferable that at least one 6-membered ring of the two 6-membered rings bonded to Het¹ forms a fused ring. It is more preferable that the two 6-membered rings bonded to Het¹ form a fused ring.

In General Formula (1), it is preferable that the 6-membered ring composed of X^{a}, X^{b}, Y^{a}, Y^{b}, Y^{c}, and a carbon atom does not form a perimidine ring. In General Formula (1), it is preferable that the 6-membered ring composed of X^{c}, X^{d}, Y^{d}, Y^{e}, Y^{f}, and a carbon atom does not form a perimidine ring.

In General Formula (1), each of the two 6-membered rings bonded to Het¹ is directly bonded to the 5-membered or 6-membered aromatic heterocyclic ring included in the aromatic heterocyclic residue represented by Het¹. The bonding positions of the two 6-membered rings in the aromatic heterocyclic ring are not limited. Examples of the bonding positions of the two 6-membered rings in the pyrrole ring, the furan ring, or the thiophene ring, which is one kind of the aromatic heterocyclic rings, include a 2-position and a 3-position, a 2-position and a 4-position, a 2-position and a 5-position, a 3-position and a 4-position, and a 3-position and a 5-position. The bonding positions of the two 6-membered rings in the pyrrole ring, the furan ring, or the thiophene ring, which is one kind of the aromatic heterocyclic rings, are preferably a 2-position and a 4-position, a 2-position and a 5-position, or a 3-position and a 4-position, preferably a 2-position and a 5-position, or a 3-position and a 4-position, and particularly preferably a 2-position and a 5-position. Examples of the bonding positions of the two 6-membered rings in the pyridine ring which is one kind of the aromatic heterocyclic rings include a 2-position and a 3-position, a 2-position and a 4-position, a 2-position and a 5-position, a 2-position and a 6-position, a 3-position and a 4-position, a 3-position and a 5-position, and a 3-position and a 6-position. The bonding positions of the two 6-membered rings in the pyridine ring which is one kind of the aromatic heterocyclic rings are preferably a 2-position and a 5-position, a 2-position and a 6-position, or a 3-position and a 5-position, preferably a 2-position and a 5-position, or a 2-position and a 6-position, and particularly preferably a 2-position and a 5-position.

The compound represented by General Formula (1) is preferably a compound represented by General Formula (2).

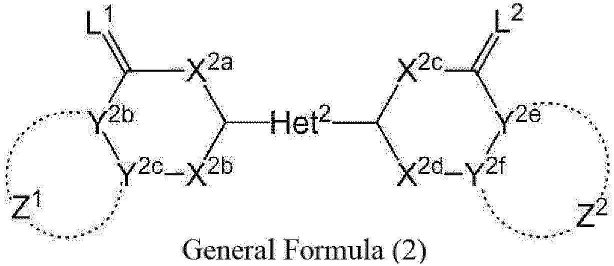

In General Formula (2), Het² represents a divalent aromatic heterocyclic residue of a 5-membered ring or a 6-membered ring, X^{2a}, X^{2b}, X^{2c}, and X^{2d} each independently represent a heteroatom, Y^{2b}, Y^{2c}, Y^{2e}, and Y^{2f} each independently represent a heteroatom or a carbon atom, two 6-membered rings bonded to Het² may each independently have a double bond, L¹ and L² each independently represent an oxygen atom, a sulfur atom, or NR^{a}, where R^{a} represents a hydrogen atom or a monovalent substituent, Z¹ represents an atomic group that is required for bonding to Y^{2b} and Y^{2c} to form a 4-membered ring to an 8-membered ring, and Z² represents an atomic group that is required for bonding to Y^{2e} and Y^{2f} to form a 4-membered ring to an 8-membered ring.

Het² in General Formula (2) has the same meaning as Het¹ in General Formula (1) described above. The preferred aspect of Het² is the same as the preferred aspect of Het¹.

X^{2a}, X^{2b}, X^{2c}, and X^{2d} in General Formula (2) respectively have the same meaning as X^{a}, X^{b}, X^{c}, and X^{d} in General Formula (1) described above. The preferred aspects of X^{2a}, X^{2b}, X^{2c}, and X^{2d} are respectively the same as the preferred aspects of X^{a}, X^{b}, X^{c}, and X^{d}. It is preferable that X^{2a} and X^{2c} are the same. It is preferable that X^{2b} and X^{2d} are the same. Regarding a combination of X^{2a}, X^{2b}, X^{2c}, and X^{2d}, it is preferable that X^{2a} and X^{2c} are an oxygen atom and X^{2b} and X^{2d} are a nitrogen atom or that X^{2a} and X^{2c} are a nitrogen atom and X^{2b} and X^{2d} are a nitrogen atom, and it is more preferable that X^{2a} and X^{2c} are an oxygen atom and X^{2b} and X^{2d} are a nitrogen atom.

Y^{2b}, Y^{2c}, Y^{2e}, and Y^{2f} in General Formula (2) respectively have the same meaning as Y^{b}, Y^{c}, Y^{e}, and Y^{f} in General Formula (1) described above. The preferred aspects of Y^{2b}, Y^{2c}, Y^{2e}, and Y^{2f} are respectively the same as the preferred aspects of Y^{b}, Y^{c}, Y^{e}, and Y^{f}.

In General Formula (2), L¹ and L² each independently represent an oxygen atom, a sulfur atom, or NR^{a}, where R^{a} represents a hydrogen atom or a monovalent substituent. Examples of the monovalent substituent represented by R^{a} include a monovalent substituent that is capable of being introduced into the above-described aromatic heterocyclic ring. L¹ and L² are each independently preferably an oxygen atom or NR^{a} and more preferably an oxygen atom. It is preferable that L¹ and L² are the same. L¹ and L² are preferably an oxygen atom.

In General Formula (2), Z¹ represents an atomic group that is required for bonding to Y^{2b} and Y^{2c} to form a 4-membered ring to an 8-membered ring. Z¹ is preferably an atomic group necessary for forming a 6-membered ring by being bonded to Y^{2b} and Y^{2c}. Examples of the 4-membered ring to the 8-membered ring include an aliphatic hydrocarbon ring (for example, a cyclohexane ring or a cyclopentane ring), an aromatic hydrocarbon ring (for example, a benzene ring or a naphthalene ring), and a heterocyclic ring (for example, a pyridine ring, a pyrrole ring, a pyridazine ring, a thiophene ring, an imidazole ring, a furan ring, a pyrazole ring, an oxazole ring, a triazole ring, a thiazol ring, or a benzo-fused ring thereof). Among the above, an aromatic hydrocarbon ring or a heterocyclic ring is preferable, an aromatic hydrocarbon ring is more preferable, and a benzene ring is particularly preferable. The ring formed from Z¹ may further have a substituent. Examples of the substituent include a monovalent substituent that is capable of being introduced into the above-described aromatic heterocyclic ring.

In General Formula (2), Z² represents an atomic group that is required for bonding to Y^{2e} and Y^{2f} to form a 4-membered ring to an 8-membered ring. Z² is preferably an atomic group necessary for forming a 6-membered ring by being bonded to Y^{2e} and Y^{2f}. Examples of the 4-membered ring to the 8-membered ring include an aliphatic hydrocarbon ring (for example, a cyclohexane ring or a cyclopentane ring), an aromatic hydrocarbon ring (for example, a benzene ring or a naphthalene ring), and a heterocyclic ring (for example, a pyridine ring, a pyrrole ring, a pyridazine ring, a thiophene ring, an imidazole ring, a furan ring, a pyrazole ring, an oxazole ring, a triazole ring, a thiazol ring, or a benzo-fused ring thereof). Among the above, an aromatic hydrocarbon ring or a heterocyclic ring is preferable, an aromatic hydrocarbon ring is more preferable, and a benzene ring is particularly preferable. The ring formed from Z² may further have a substituent. Examples of the substituent include a monovalent substituent that is capable of being introduced into the above-described aromatic heterocyclic ring.

In General Formula (2), Z¹ and Z² may form the same ring or rings different from each other. Z¹ and Z² preferably form the same ring, more preferably form a 6-membered ring, and particularly preferably form a benzene ring.

Specific examples of the ring bonded to Het² in General Formula (2) are shown below. However, the ring bonded to Het² is not limited to the specific examples shown below. "*" indicated in the following chemical structures represents a bonding site to Het².

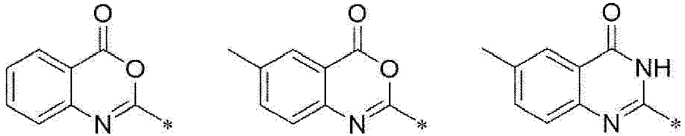

In General Formula (2), examples of the ring bonded to Het² include the rings described in paragraph 0026 to paragraph 0027 of JP5236297B. The description of the above publication is incorporated in the present specification by reference.

The compound represented by General Formula (2) is preferably a compound represented by General Formula (3).

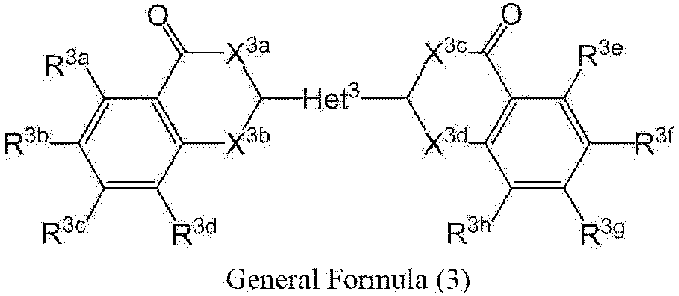

In General Formula (3), Het³ represents a divalent aromatic heterocyclic residue of a 5-membered ring or a 6-membered ring, X^{3a}, X^{3b}, X^{3c}, and X^{3d} each independently represent a heteroatom, X^{3a} or X^{3b} may form a double bond with a carbon atom in a ring bonded to Het³, X^{3c} or X^{3d} may form a double bond with a carbon atom in a ring bonded to Het³, and R^{3a}, R^{3b}, R^{3c}, R^{3d}, R^{3e}, R^{3f}, R^{3g}, and R^{3h} each independently represent a hydrogen atom or a monovalent substituent.

Het³ in General Formula (3) has the same meaning as Het¹ in General Formula (1) described above. The preferred aspect of Het³ is the same as the preferred aspect of Het¹.

X^{3a}, X^{3b}, X^{3c}, and X^{3d} in General Formula (3) respectively have the same meaning as X^{a}, X^{b}, X^{c}, and X^{d} in General Formula (1) described above. The preferred aspects of X^{3a}, X^{3b}, X^{3c}, and X^{3d} are respectively the same as the preferred aspects of X^{a}, X^{b}, X^{c}, and X^{d}. It is preferable that X^{3a} and X^{3c} are the same. It is preferable that X^{3b} and X^{3d} are the same. Regarding a combination of X^{3a}, X^{3b}, X^{3c}, and X^{3d}, it is preferable that X^{3a} and X^{3c} are an oxygen atom and X^{3b} and X^{3d} are a nitrogen atom or that X^{3a} and X^{3c} are a nitrogen atom and X^{3b} and X^{3d} are a nitrogen atom, and it is more preferable that X^{3a} and X^{3c} are an oxygen atom and X^{3b} and X^{3d} are a nitrogen atom.

In General Formula (3), R^{3a}, R^{3b}, R^{3c}, R^{3d}, R^{3e}, R^{3f}, R^{3g}, and R^{3h} each independently represent a hydrogen atom or a monovalent substituent. Examples of the monovalent substituent represented by R^{3a}, R^{3b}, R^{3c}, R^{3d}, R^{3e}, R^{3f}, R^{3g}, and R^{3h} include a monovalent substituent that is capable of being introduced into the above-described aromatic heterocyclic ring. Any two of R^{3a}, R^{3b}, R^{3c}, and R^{3d} may be bonded to each other to form a ring. Any two of R^{3e}, R^{3f}, R^{3g}, and R^{3h} may be bonded to each other to form a ring. R^{3a}, R^{3b}, R^{3c}, R^{3d}, R^{3e}, R^{3f}, R^{3g}, and R^{3h} are each independently preferably a hydrogen atom, an alkyl group having 1 to 10 carbon atoms, an alkoxy group having 1 to 10 carbon atoms, or a hydroxy group, more preferably a hydrogen atom, an alkyl group having 1 to 10 carbon atoms, or an alkoxy group having 1 to 10 carbon atoms, still more preferably a hydrogen atom or an alkyl group having 1 to 10 carbon atoms, and particularly preferably a hydrogen atom.

The compound represented by General Formula (3) is preferably a compound represented by General Formula (4).

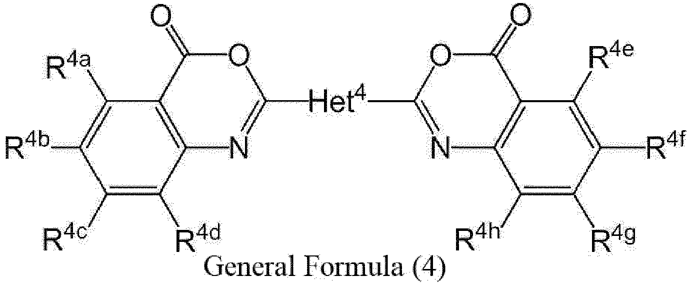

In General Formula (4), Het⁴ represents a divalent aromatic heterocyclic residue of a 5-membered ring or a 6-membered ring, and R^{4a}, R^{4b}, R^{4c}, R^{4d}, R^{4e}, R^{4f}, R^{4g}, and R^{4h} each independently represent a hydrogen atom or a monovalent substituent.

Het⁴ in General Formula (4) has the same meaning as Het¹ in General Formula (1) described above. The preferred aspect of Het⁴ is the same as the preferred aspect of Het¹.

R^{4a}, R^{4b}, R^{4c}, R^{4d}, R^{4e}, R^{4f}, R^{4g}, and R^{4h} in General Formula (4) respectively have the same meanings as R^{3a}, R^{3b}, R^{3c}, R^{3d}, R^{3e}, R^{3f}, R^{3g}, and R^{3h} in General Formula (3). The preferred aspects of R^{4a}, R^{4b}, R^{4c}, R^{4d}, R^{4e}, R^{4f}, R^{4g}, and R^{4h} are respectively the same as the preferred aspects of R^{3a}, R^{3b}, R^{3c}, R^{3d}, R^{3e}, R^{3f}, R^{3g}, and R^{3h}.

The compound represented by General Formula (4) is preferably a compound represented by General Formula (5).

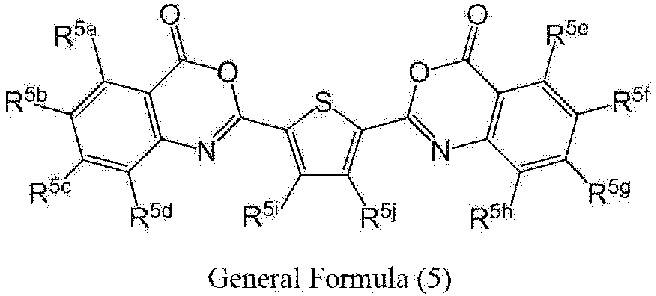

In General Formula (5), R^{5a}, R^{5b}, R^{5c}, R^{5d}, R^{5e}, R^{5f}, R^{5g}, and R^{5h} each independently represent a hydrogen atom or a monovalent substituent, and R⁵ⁱ and R^{5j} each independently represent a hydrogen atom or a monovalent substituent.

R^{5a}, R^{5b}, R^{5c}, R^{5d}, R^{5e}, R^{5f}, R^{5g}, and R^{5h} in General Formula (5) respectively have the same meanings as R^{3a}, R^{3b}, R^{3c}, R^{3d}, R^{3e}, R^{3f}, R^{3g}, and R^{3h} in General Formula (3). The preferred aspects of R^{5a}, R^{5b}, R^{5c}, R^{5d}, R^{5e}, R^{5f}, R^{5g}, and R^{5h} are respectively the same as the preferred aspects of R^{3a}, R^{3b}, R^{3c}, R^{3d}, R^{3e}, R^{3f}, R^{3g}, and R^{3h}.

In General Formula (5), R⁵ⁱ and R^{5j} each independently represent a hydrogen atom or a monovalent substituent. Examples of the monovalent substituent represented by R⁵ⁱ and R^{5j} include a monovalent substituent that is capable of being introduced into the above-described aromatic heterocyclic ring. R⁵ⁱ and R^{5j} may be bonded to each other to form a ring. R⁵ⁱ and R^{5j} are each independently preferably a hydrogen atom, an alkyl group having 1 to 10 carbon atoms, an alkoxy group having 1 to 10 carbon atoms, or a hydroxy group, more preferably a hydrogen atom or an alkoxy group having 1 to 10 carbon atoms, and particularly preferably a hydrogen atom.

Specific examples of the compound included in the compound represented by each of the general formulae described above are shown below. However, the aspect of the compound represented by each of the general formulae described above is not limited to the compounds shown below.

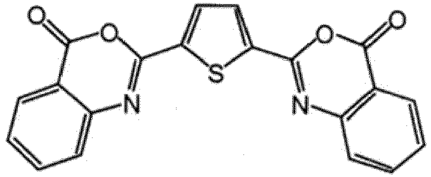

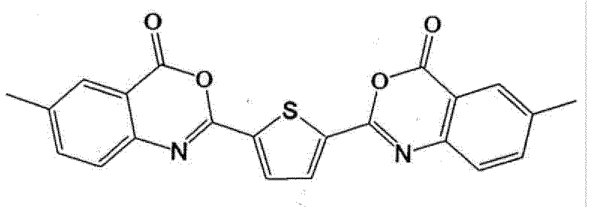

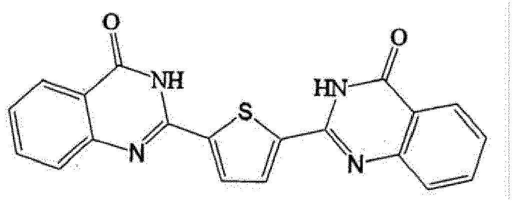

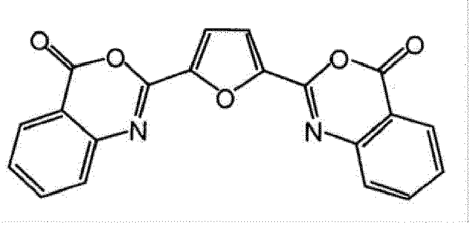

Examples of the compound included in the compound represented by each of the general formulae described above also include the compounds described in paragraph 0047 to paragraph 0056 of JP5236297B, the compounds described in paragraph 0046 of WO2018/180929A, and the compounds described in paragraph 0070 to paragraph 0075 of JP2010-132846A. The descriptions of the above publications are incorporated in the present specification by reference.

### [Absorption wavelength]

The compound according to one embodiment of the present disclosure preferably has an absorption wavelength in a wavelength range of 410 nm to 420 nm. The compound having an absorption wavelength in a wavelength range of 410 nm to 420 nm can impart the absorbability of blue light to various articles, in combination with, for example, a polymer compound. The absorption wavelength is measured according to absorption spectrophotometry.

### [Production method]

A method of forming the chemical structure represented by General Formula (1) is not limited. As the method of forming the chemical structure represented by General Formula (1), a known method can be used. As the method of forming the chemical structure represented by General Formula (1), it is possible to use, for example, the method described in JP5236297B or WO2018/180929A. The descriptions of the above publications are incorporated in the present specification by reference.

For example, the following compound can be produced through an intramolecular condensation reaction of the following synthetic intermediate A obtained by a reaction of anthranilic acid with 2,5-thiophenedicarbonyl dichloride (also known as 2,5-bis(chlorocarbonyl)thiophene).

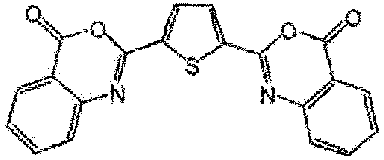

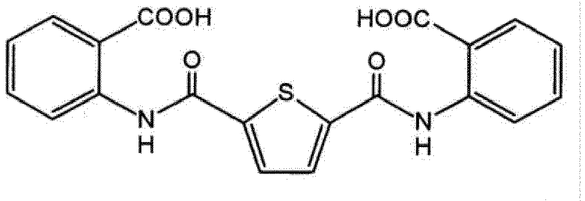

### (Synthetic intermediate A)

It is preferable that the production method for the compound according to one embodiment of the present disclosure includes a step of washing the compound represented by General Formula (1) with water (hereinafter, referred to as a "washing step"). In a case of washing the compound represented by General Formula (1) with water, it is possible to reduce the content of ions (for example, chloride ions, sodium ions, aluminum ions, iron ions, and calcium ions) in the compound. Examples of the preferred water that is used in the washing step include pure water and ultrapure water. The using amount of water per washing is preferably 10 L or more with respect to 1 kg of the compound (specifically, the compound represented by General Formula (1)). As the washing solvent, water and a water-soluble solvent other than the water may be used in combination. Examples of the water-soluble solvent include acetone, methanol, ethanol, propanol, isopropanol, acetonitrile, tetrahydrofuran, dimethylformamide, and dimethylacetamide. In the washing step, one kind or two or more kinds of water-soluble solvents may be used. In the washing step, a mixture of two or more kinds of water-soluble solvents may be used. The water-soluble solvent is preferably at least one selected from the group consisting of acetone, methanol, ethanol, propanol, isopropanol, acetonitrile, tetrahydrofuran, dimethylformamide, and dimethylacetamide. The water-soluble solvent is also preferably at least one selected from the group consisting of acetone and methanol. For example, water and acetone are preferable as a combination of the washing solvents. For example, water, acetone, and methanol are also preferable as a combination of the washing solvents. The washing step may include washing the compound represented by General Formula (1) with each of water and a water-soluble solvent other than the water. The washing step may include washing the compound represented by General Formula (1) with water and a mixture containing a water-soluble solvent other than the water. The number of washings in the washing step is preferably 2 or more. From the viewpoint of reducing the residual solvent, water, acetone, methanol, ethanol, or acetonitrile is preferably used in the final washing.

### [Use application]

For example, the compound according to one embodiment of the present disclosure can be used as a component of various articles together with a polymer compound. Examples of the article include a composition described below, a lens for spectacles, an optical lens, and an optical film. However, the article in which the compound according to one embodiment of the present disclosure is used is not limited to the above article. In addition, the compound according to one embodiment of the present disclosure can be used in combination with, for example, a polymer compound to impart the absorbability of ultraviolet rays or blue light to various articles.

### <Composition>

A composition according to one embodiment of the present disclosure contains a compound represented by General Formula (1) and a polymer compound, in which a content of a chloride ion is less than 1.5 ppm with respect to a total mass of the composition. According to the above embodiment, a composition having less redness is provided.

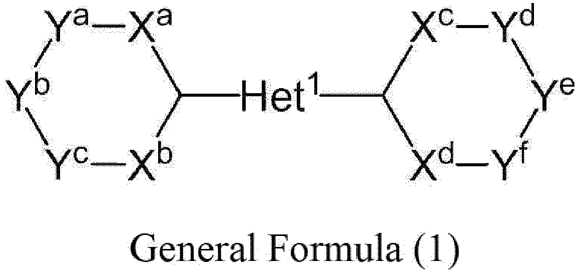

In General Formula (1), Het¹ represents a divalent aromatic heterocyclic residue of a 5-membered ring or a 6-membered ring, X^{a}, X^{b}, X^{c}, and X^{d} each independently represent a heteroatom, Y^{a}, Y^{b}, Y^{c}, Y^{d}, Y^{e}, and Y^{f} each independently represent a heteroatom or a carbon atom, and two 6-membered rings bonded to Het¹ each independently may have a double bond.

The reason why the composition according to one embodiment of the present disclosure exhibits the above-described effect is presumed as follows. It is conceived that the coloration of the composition containing the compound represented by General Formula (1) and a polymer compound occurs due to the hydrolysis of the compound represented by General Formula (1), which is caused by ions, particularly chloride ions, as described in the section of the "Compound" described above. It is conceived that the compound represented by General Formula (1) is hydrolyzed due to the influence of the chloride ion and the influence of a hydrolyzate of the polymer compound, which results in an increase in redness. On the other hand, according to the composition according to one embodiment of the present disclosure, since such hydrolysis as described above can be suppressed by setting the content of chloride ions to less than 1.5 ppm, redness can be reduced.

### [Compound]

The composition according to one embodiment of the present disclosure contains a compound represented by General Formula (1). The aspect of the compound represented by General Formula (1) is as described in the section of "Compound" described above. The preferred aspect of the compound represented by General Formula (1) is the same as the preferred aspect of the compound represented by General Formula (1) described in the section of "Compound" described above.

The compound represented by General Formula (1) is preferably a compound represented by General Formula (2).

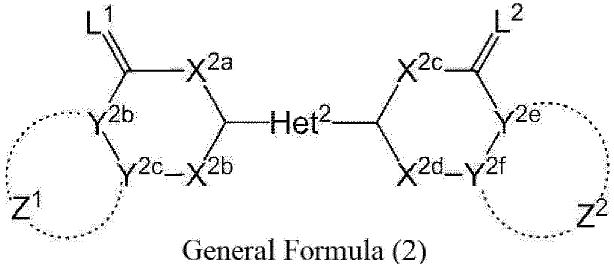

In General Formula (2), Het² represents a divalent aromatic heterocyclic residue of a 5-membered ring or a 6-membered ring, X^{2a}, X^{2b}, X^{2c}, and X^{2d} each independently represent a heteroatom, Y^{2b}, Y^{2c}, Y^{2e}, and Y^{2f} each independently represent a heteroatom or a carbon atom, two 6-membered rings bonded to Het² may each independently have a double bond, L¹ and L² each independently represent an oxygen atom, a sulfur atom, or NR^{a}, where R^{a} represents a hydrogen atom or a monovalent substituent, Z¹ represents an atomic group that is required for bonding to Y^{2b} and Y^{2c} to form a 4-membered ring to an 8-membered ring, and Z² represents an atomic group that is required for bonding to Y^{2e} and Y^{2f} to form a 4-membered ring to an 8-membered ring.

The aspect of the compound represented by General Formula (2) is as described in the section of "Compound" described above. The preferred aspect of the compound represented by General Formula (2) is the same as the preferred aspect of the compound represented by General Formula (2) described in the section of "Compound" described above.

The compound represented by General Formula (2) is preferably a compound represented by General Formula (3).

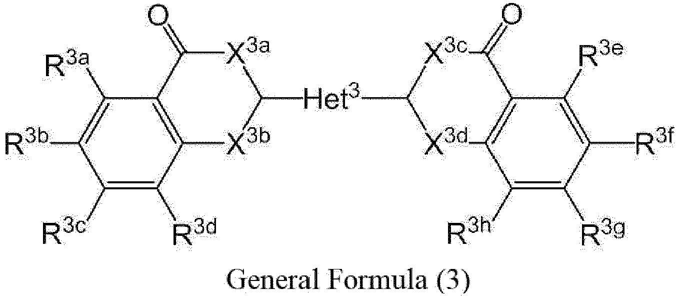

In General Formula (3), Het³ represents a divalent aromatic heterocyclic residue of a 5-membered ring or a 6-membered ring, X^{3a}, X^{3b}, X^{3c}, and X^{3d} each independently represent a heteroatom, X^{3a} or X^{3b} may form a double bond with a carbon atom in a ring bonded to Het³, X^{3c} or X^{3d} may form a double bond with a carbon atom in a ring bonded to Het³, and R^{3a}, R^{3b}, R^{3c}, R^{3d}, R^{3e}, R^{3f}, R^{3g}, and R^{3h} each independently represent a hydrogen atom or a monovalent substituent.

The aspect of the compound represented by General Formula (3) is as described in the section of "Compound" described above. The preferred aspect of the compound represented by General Formula (3) is the same as the preferred aspect of the compound represented by General Formula (3) described in the section of "Compound" described above.

The compound represented by General Formula (3) is preferably a compound represented by General Formula (4).

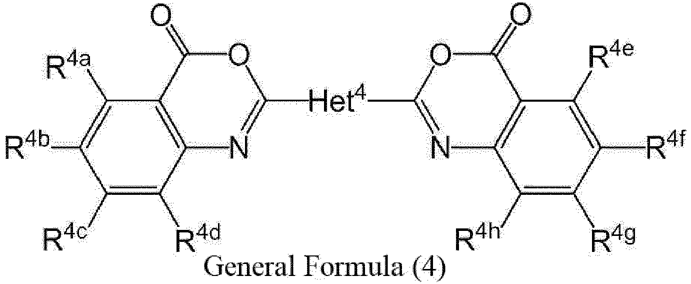

In General Formula (4), Het⁴ represents a divalent aromatic heterocyclic residue of a 5-membered ring or a 6-membered ring, and R^{4a}, R^{4b}, R^{4c}, R^{4d}, R^{4e}, R^{4f}, R^{4g}, and R^{4h} each independently represent a hydrogen atom or a monovalent substituent.

The aspect of the compound represented by General Formula (4) is as described in the section of "Compound" described above. The preferred aspect of the compound represented by General Formula (4) is the same as the preferred aspect of the compound represented by General Formula (4) described in the section of "Compound" described above.

The compound represented by General Formula (4) is preferably a compound represented by General Formula (5).

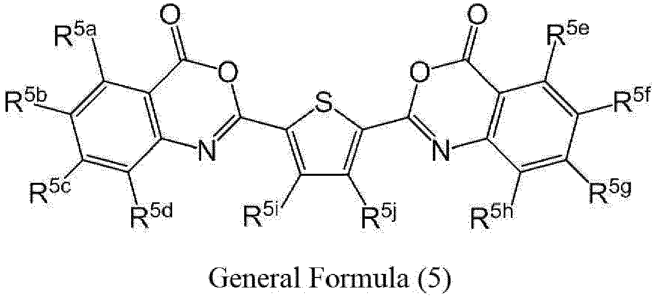

In General Formula (5), R^{5a}, R^{5b}, R^{5c}, R^{5d}, R^{5e}, R^{5f}, R^{5g}, and R^{5h} each independently represent a hydrogen atom or a monovalent substituent, and R⁵ⁱ and R^{5j} each independently represent a hydrogen atom or a monovalent substituent.

The aspect of the compound represented by General Formula (5) is as described in the section of "Compound" described above. The preferred aspect of the compound represented by General Formula (5) is the same as the preferred aspect of the compound represented by General Formula (5) described in the section of "Compound" described above.

The composition according to one embodiment of the present disclosure may contain one kind or two or more kinds of compounds represented by General Formula (1).

From the viewpoint of sufficiently absorbing light, the content of the compound represented by General Formula (1) is preferably 0.01% by mass or more, more preferably 0.5% by mass or more, and particularly preferably 1% by mass or more, with respect to the total mass of the composition. From the viewpoint of maintaining the physical characteristics of the composition, the content of the compound represented by General Formula (1) is preferably 10% by mass or less, more preferably 5% by mass or less, and particularly preferably 3% by mass or less, with respect to the total mass of the composition.

### [Polymer compound]

The composition according to one embodiment of the present disclosure contains a polymer compound. Examples of the role of the polymer compound include a role as a dispersing agent of the compound represented by General Formula (1) and a role in molding the composition. In addition, according to the polymer compound, it is possible to impart, for example, various functions to the composition according to one embodiment of the present disclosure. The polymer compound that is applied to the composition according to one embodiment of the present disclosure does not include the compound represented by General Formula (1) described above.

The weight-average molecular weight of the polymer compound is preferably 10,000 or more, more preferably 20,000 or more, and particularly preferably 30,000 or more. The upper limit of the weight-average molecular weight of the polymer compound is not limited. The weight-average molecular weight of the polymer compound is preferably 1,000,000 or less, more preferably 500,000 or less, and particularly preferably 200,000 or less. The weight-average molecular weight is a polystyrene-equivalent molecular weight measured by gel permeation chromatography (GPC).

Examples of the polymer compound include polyester (for example, polyethylene terephthalate (PET), polyethylene naphthalate (PEN), or fluorene-based polyester), polycarbonate, polyethylene, polypropylene, an acrylic resin, cyclic polyolefin (for example, a cycloolefin polymer (COP)) or a cyclic olefin copolymer (COC)), an epoxy resin, polyurethane, polythiourethane, polyimide, polyamide, and a fluororesin. In addition, examples of the polymer compound include the polymer substances described in paragraph 0075 to paragraph 0076 of JP5236297B and the resins described in paragraph 0030, paragraph 0031, and paragraph 0033 of WO2018/180929A. The descriptions of the above publications are incorporated in the present specification by reference. From the viewpoint of processability, the polymer compound may be a thermoplastic polymer compound. The thermoplastic polymer compound may be appropriately selected from the above-described polymer compounds and known thermoplastic polymer compounds. Specific examples of the thermoplastic polymer compound include polyethylene terephthalate, polycarbonate, an acrylic resin, and polyamide. The polymer compound may be a polymer. The polymer may be a homopolymer or a copolymer.

The polymer compound is preferably at least one selected from the group consisting of polyester, polycarbonate, polyethylene, polypropylene, an acrylic resin, cyclic polyolefin, an epoxy resin, polyurethane, polythiourethane, polyimide, polyamide, and a fluororesin, more preferably polyester, polycarbonate, an acrylic resin, polyurethane, or polythiourethane, and particularly preferably polyester or polycarbonate.

The composition according to one embodiment of the present disclosure may contain one kind or two or more kinds of polymer compounds.

From the viewpoint of maintaining the physical characteristics of the polymer compound, the content of the polymer compound is preferably 90% by mass or more, more preferably 95% by mass or more, and particularly preferably 97% by mass or more, with respect to the total mass of the composition. The upper limit of the content of the polymer compound is not limited. The content of the polymer compound is preferably less than 100% by mass and more preferably 99% by mass or less with respect to the total mass of the composition.

The ratio (M2/M1) of the content (M2) of the compound represented by General Formula (1) to the content (M1) of the polymer compound is preferably 0.0001 or more, more preferably 0.005 or more, and particularly preferably 0.01 or more, in terms of mass. The ratio (M2/M1) of the content (M2) of the compound represented by General Formula (1) to the content (M1) of the polymer compound is preferably 0.1 or less, more preferably 0.05 or less, and particularly preferably 0.03 or less.

### [Ion]

### (Chloride ion)

The content of chloride ions (in terms of mass) is less than 1.5 ppm with respect to the total mass of the composition. In a certain embodiment, the content of chloride ions (in terms of mass) is preferably less than 1.2 ppm and more preferably less than 1 ppm with respect to the total mass of the composition. In a case where the content of chloride ions is less than 1.5 ppm, redness can be reduced. In addition, haze can be reduced by reducing the content of chloride ions in the composition. The lower limit of the content of chloride ions is not limited. For example, the content of chloride ions (in terms of mass) may be 0 ppm or more with respect to the total mass of the composition. The content of chloride ions is measured according to the measuring method described in the section of "Compound" described above.

### (Sodium ion)

In a certain embodiment, it is preferable to reduce not only the content of chloride ions but also the content of sodium ions. In a case of reducing the content of sodium ions in addition to the chloride ions, it is possible to further reduce redness. In addition, in a case of reducing the content of sodium ions in addition to the chloride ions, it is possible to further reduce haze. The content of sodium ions (in terms of mass) is preferably less than 1.5 ppm, more preferably less than 1.2 ppm, and particularly preferably less than 1 ppm, with respect to the total mass of the composition. The lower limit of the content of sodium ions is not limited. For example, the content of sodium ions (in terms of mass) may be 0 ppm or more with respect to the total mass of the composition. The content of sodium ions is measured according to the measuring method described in the section of "Compound" described above.

From the viewpoint of suppressing an increase in redness and reducing haze, it is preferable to reduce the total content of chloride ions and sodium ions. In a certain embodiment, the total content of chloride ions and sodium ions is preferably less than 3 ppm, more preferably less than 2.5 ppm, and particularly preferably less than 2 ppm, with respect to the total mass of the composition. The lower limit of the total content of chloride ions and sodium ions is not limited. The total content of chloride ions and sodium ions may be determined in a range of 0 ppm or more with respect to the total mass of the composition.

Examples of the method of reducing the content of each of the above-described ions include a method of producing a composition using a raw material having high purity. For example, by using a highly pure compound represented by General Formula (1) as a raw material, the content of each of the above-described ions can be reduced.

### [Another component]

The composition according to one embodiment of the present disclosure may contain a component other than the above-described components (hereinafter, referred to as "another component"), as necessary. Examples of the other component include a filler, a plasticizer, a surfactant, an adhesion accelerator, an antioxidant, an aggregation preventing agent, a processing stabilizer, a compatibilizer, a dispersing agent, an antifoaming agent, a dye, a pigment, an infrared absorbing agent, a fragrance, an inorganic substance, and an ultraviolet absorbing agent. The plasticizer is not limited, and a known plasticizer can be used. Examples of the plasticizer include a phthalic acid ester (for example, dimethyl phthalate, diethyl phthalate, diisopropyl phthalate, dibutyl phthalate, diisobutyl phthalate, dihexyl phthalate, dicyclohexyl phthalate, or diphenyl phthalate), a phosphoric acid ester (for example, trimethyl phosphate, triethyl phosphate, tributyl phosphate, triphenyl phosphate, or tricresyl phosphate), a trimellitic acid ester (for example, tributyl trimellitate or tris(2-ethylhexyl)trimellitate), and a fatty acid esters (for example, dimethyl adipate, diethyl adipate, dipropyl adipate, diisopropyl adipate, dibutyl adipate, diisobutyl adipate, dimethyl dodecanoate, dibutyl maleate, or ethyl oleate). Examples of the antioxidant include a phenol-based antioxidant, a hydroquinone-based antioxidant, a phosphorus-based antioxidant, and a hydroxylamine-based antioxidant. Examples of the phenol-based antioxidant and the hydroquinone-based antioxidant include 2,6-di-tert-butyl-4-methylphenol, 4,4'-thiobis-(6-tert-butyl-3-methylphenol), 1,1'-bis(4-hydroxyphenyl)cyclohexane, 2,2'-methylenebis(4-ethyl-6-tert-butylphenol), 2,5-di-tert-butylhydroquinone, and pentaerythrityl-tetrakis[3-(3,5-di-tert-butyl-4-hydroxyphenyl)propionate]. Examples of the phosphorus-based antioxidant include a phosphite-based antioxidant (for example, tris(4-methoxy-3,5-diphenyl)phosphite, tris(nonylphenyl)phosphite, tris (2,4-di-tert-butylphenyl)phosphite, bis(2,6-di-tert-butyl-4-methylphenyl)pentaeristol diphosphite, and bis(2,4-di-tert-butylphenyl)pentaerythritol diphosphite). Examples of the hydroxylamine-based antioxidant include N,N-dioctadecylhydroxylamine and N,N-dibenzylhydroxylamine. Examples of the ultraviolet absorbing agent include a triazine compound, a benzotriazole compound, a benzophenone compound, a salicylic acid compound, and a metal oxide particle. The ultraviolet absorbing agent may be a polymer having an ultraviolet absorbing structure. Examples of the polymer including an ultraviolet absorbing structure include an acrylic resin containing a monomer unit derived from an acrylic acid ester compound containing at least a part of the structure such as a triazine compound, a benzotriazole compound, a benzophenone compound, or a salicylic acid compound. A triazine compound is particularly preferable.

### [Form]

The form of the composition according to one embodiment of the present disclosure is not limited. The form of the composition may be determined, for example, depending on the use application. Examples of the form of the composition include an article including a curved surface (for example, a lens), a flat plate (including a film and a sheet), a powder material, and an amorphous article. The composition may be a pellet. The composition can be molded by various molding methods. Examples of the molding method include a melt extrusion method and an injection molding method. The composition may be a molded body.

### [Production method]

The production method for the composition according to one embodiment of the present disclosure is not limited. For example, a composition can be produced by mixing the compound represented by General Formula (1) with a polymer compound. Preferred examples of the production method for the composition according to one embodiment of the present disclosure include a melt extrusion method. For example, it is possible to produce a composition containing the compound represented by General Formula (1) and a polymer compound by charging the compound represented by General Formula (1) and a polymer compound into an extruder and then kneading them under heating conditions to obtain a melt, which is subsequently extruded. Further, after producing a pellet using the composition obtained according to the above-described method, it is also possible to produce a composition containing the pellet and a polymer compound by charging the compound represented by General Formula (1) and a polymer compound into an extruder and subsequently kneading them under heating conditions to obtain a melt, which is extruded. Examples of the production method for the composition according to one embodiment of the present disclosure also include a method using polymerization of a polymerizable compound contained in a mixture of the compound represented by General Formula (1) and a polymerizable compound (a raw material of a polymer compound).

### [Use application]

The composition according to one embodiment of the present disclosure can be used for various use applications. Examples of the use application of the composition include various molded bodies (for example, a lens for spectacles, an optical lens, and an optical film). However, the use application of the composition is not limited to the above-described use applications.

### (Lens for spectacles)

In a certain embodiment, the composition is preferably for a lens for spectacles. According to the above embodiment, a lens for spectacles, which has less redness, is provided.

The lens for spectacles is one kind of molded body of the composition according to one embodiment of the present disclosure. That is, the lens for spectacles contains the compound represented by General Formula (1) and the polymer compound.

Layers having various functions may be arranged on the surface of the lens for spectacles. Examples of the function of the layer include the prevention of scratches or stains and the prevention of reflection of light.

The lens for spectacles may include the protective layer described in paragraph 0074 to paragraph 0094 of WO2018/180929A.

The shape of the lens for spectacles is not limited. The shape of the lens for spectacles may be determined, for example, according to the design of the spectacles of interest.

The lens for spectacles can be manufactured, for example, by using a known molding method. For example, the lens for spectacles can be manufactured by molding the composition according to one embodiment of the present disclosure into a pellet shape by a melt extrusion method and then molding the obtained pellet into a lens shape by an injection molding method. However, the manufacturing method for the lens for spectacles is not limited to the above method.

The lens for spectacles can be used as spectacles by being attached to any spectacle frame. As the manufacturing method for the spectacles, a known method can be used.

Since the compound represented by General Formula (1) is contained, the lens for spectacles can reduce the transmittance of blue light. For this reason, the lens for spectacles can be used, for example, as a lens for spectacles having a function called "blue light cutting".

### (Optical lens)

In a certain embodiment, the use application of the composition is preferably for an optical lens. According to the above embodiment, an optical lens having less redness is provided.

The optical lens is one kind of molded body of the composition according to one embodiment of the present disclosure. That is, the optical lens contains the compound represented by General Formula (1) and the polymer compound.

The optical lens may include the protective layer described in paragraph 0074 to paragraph 0094 of WO2018/180929A.

The shape of the optical lens is not limited. The shape of the optical lens may be determined, for example, according to the use application.

The optical lens can be manufactured, for example, by using a known molding method. For example, the optical lens can be manufactured by molding the composition according to one embodiment of the present disclosure into a pellet shape by a melt extrusion method and then molding the obtained pellet into a lens shape by an injection molding method. However, the manufacturing method for the optical lens is not limited to the above method.

The optical lens can be used, for example, as a lens of an optical device. Examples of the optical device include a camera, a television camera, a telescope, and a microscope.

### (Optical film]

In a certain embodiment, the use application of the composition is preferably for an optical film. According to the above embodiment, an optical film having less redness is provided.

The optical film is one kind of molded body of the composition according to one embodiment of the present disclosure. That is, the optical film contains the compound represented by General Formula (1) and the polymer compound.

Layers having various functions may be arranged on the surface of the optical film. Examples of the function of the layer include the prevention of scratches or stains and the prevention of reflection of light. An adhesive layer may be arranged on the surface of the optical film in order to improve the adhesiveness between the optical film and another member.

The optical film may include the protective layer described in paragraph 0074 to paragraph 0094 of WO2018/180929A.

The shape of the optical film is not limited. The shape of the optical film may be determined, for example, according to the use application.

The optical film can be manufactured, for example, by using a known molding method. For example, the optical film can be manufactured by molding the composition according to one embodiment of the present disclosure into a film shape by a melt extrusion method. In addition, the optical film can also be manufactured by molding the composition according to one embodiment of the present disclosure into a pellet shape by a melt extrusion method and then molding the obtained pellet into a film shape by a melt extrusion method. However, the manufacturing method for the optical film is not limited to the above method.

The optical film can be used, for example, by being arranged on a surface of a video display unit in an electronic device. Examples of the electronic device include a smartphone, a tablet terminal, a display device (for example, a liquid crystal display device), and a personal computer. In addition, since the compound represented by General Formula (1) is contained, the optical film can reduce the transmittance of blue light. For this reason, the optical film can be used, for example, as an optical film having a function called "blue light cutting".

### (Another use application)

The composition according to one embodiment of the present disclosure can be suitably used for use applications in which exposure to light including, for example, sunlight or ultraviolet rays is assumed. Specific examples of the use application include a glass substitute and a surface coating material thereof, a window glass for a house, a facility, transport equipment, or the like, a coating material for lighting glass or light source protective glass, a window film for a house, a facility, transport equipment or the like, an interior/exterior material for a house, a facility, transport equipment or the like, and an interior/exterior paint, a coating film formed from the paint, an alkyd resin lacquer paint and a coating film formed from the paint, an acrylic lacquer paint and a coating film formed from the paint, a light source member that emit ultraviolet rays, such as a fluorescent lamp or a mercury lamp, precision machinery, a parts for electronic and electrical equipment, a material for blocking electromagnetic waves or the like, generated from various displays, a container or packaging material for a food, a chemical, a medicine, or the like, a bottle, a box, a blister, a cup, a special package, a compact disc coat, or a sheet or film material for agriculture, an anti-fading agent for printed matter, dyed matter, a dye or pigment, or the like, a protective film for a polymer support (for example, for a plastic component such as a machine component or an automobile component), a printed matter overcoat, an inkjet medium coating film, a laminate matte, an optical light film, a safety glass/front glass interlayer, an electrochromic/photochromic use application, an overlaminate film, a solar heat control film, a cosmetic such as a sunscreen cream, a shampoo, a rinse, a hairdressing agent, or the like, a textile product for clothing such as sportswear, a stocking, or hat, and a household interior material such as a textile, a curtain, a rug, or wallpaper, a medical instrument such as a plastic lens, a contact lens, or an artificial eye, an optical article such as an optical filter, a backlight display film, a prism, a mirror, or a photographic material, a mold film, a transfer type sticker, an anti-scribble film, stationery such as tape or an ink, and a sign board, a sign device, and a surface coating material thereof.

### [Another embodiment]

A composition according to another embodiment of the present disclosure contains the compound represented by General Formula (1) and a polymer compound, and it has an absolute fluorescence quantum yield of less than 0.1. According to the above embodiment, a composition having excellent light resistance is provided since the fluorescence is reduced. Hereinafter, the composition according to the above embodiment will be described.

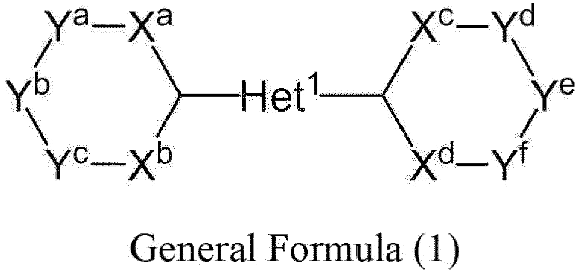

In General Formula (1), Het¹ represents a divalent aromatic heterocyclic residue of a 5-membered ring or a 6-membered ring, X^{a}, X^{b}, X^{c}, and X^{d} each independently represent a heteroatom, Y^{a}, Y^{b}, Y^{c}, Y^{d}, Y^{e}, and Y^{f} each independently represent a heteroatom or a carbon atom, and two 6-membered rings bonded to Het¹ each independently may have a double bond.

The aspect of the compound represented by General Formula (1) is as described in the section of "Compound" described above. The preferred aspect of the compound represented by General Formula (1) is the same as the preferred aspect of the compound represented by General Formula (1) described in the section of "Compound" described above.

The absolute fluorescence quantum yield is measured using a known emission quantum yield measuring device. A device capable of measuring the absolute fluorescence quantum yield can be obtained from, for example, Hamamatsu Photonics K.K. or JASCO Corporation.

Examples of the method of reducing the absolute fluorescence quantum yield of a composition include the use of an ultraviolet absorbing agent, the reduction of the content of metal ions, and the use of a fluorescence quencher.

From the viewpoint of reducing the absolute fluorescence quantum yield, the composition according to another embodiment of the present disclosure preferably contains an ultraviolet absorbing agent. The ultraviolet absorbing agent referred to here does not include the compound represented by General Formula (1). Examples of the ultraviolet absorbing agent include a benzotriazole-based ultraviolet absorbing agent (that is, an ultraviolet absorbing agent having a benzotriazole skeleton) and a benzophenone-based ultraviolet absorbing agent (that is, an ultraviolet absorbing agent having a benzophenone skeleton). The ultraviolet absorbing agent preferably has an absorption wavelength in a wavelength range of 400 nm or less. In addition, in a case where the composition is used for a molded body, a layer containing an ultraviolet absorbing agent may be formed on the surface of the molded body.

From the viewpoint of reducing the absolute fluorescence quantum yield, it is preferable that the content of metal ions in the composition according to another embodiment of the present disclosure is small. Examples of the metal ion include a sodium ion, an aluminum ion, an iron ion, and a calcium ion. Examples of the method of reducing the content of each of the above-described ions include a method of producing a composition using a raw material having high purity. For example, by using a highly pure compound represented by General Formula (1) as a raw material, the content of each of the above-described ions can be reduced. For example, the content of sodium ions in the composition according to another embodiment of the present disclosure is preferably within the range of the content of sodium ions in the composition in the section of "Ion" described above.

From the viewpoint of reducing the absolute fluorescence quantum yield, the composition according to another embodiment of the present disclosure preferably contains a fluorescence quencher. Examples of the fluorescence quencher include Kayaclean (Nippon Kayaku Co., Ltd.).

As the polymer compound, it is possible to use, for example, the polymer compound described in the section of "Polymer compound" described above. The polymer compound is preferably a thermoplastic polymer compound. The thermoplastic polymer compound may be appropriately selected from, for example, the polymer compound described in the section of "Polymer compound" described above and a known thermoplastic polymer compound. Specific examples of the thermoplastic polymer compound include polyethylene terephthalate, polycarbonate, an acrylic resin, and polyamide.

The content of chloride ions in the composition according to another embodiment of the present disclosure is preferably within the range of the content of chloride ions in the composition described in the section of "Ions" described above.

The total content of chloride ions and sodium ions in the composition according to another embodiment of the present disclosure is preferably within the range of the total content of chloride ions and sodium ions in the composition described in the section of "Ion" described above.

The composition according to another embodiment of the present disclosure may contain the component described in the section of "Another component" described above.

Regarding the form, the production method, and the use application of the composition according to another embodiment of the present disclosure, the respective items of the above-described "form", "production method", and "use application" can be referred to. Examples

Hereinafter, the present disclosure will be described in detail according to Examples. However, the present disclosure is not limited to the following Examples.

### <Synthesis of compound (1)>

Anthranilic acid (1.3 g) was dissolved in dimethylacetamide (12 mL), and 2,5-thiophenedicarbonyl dichloride (1.0 g) was added thereto at room temperature. The obtained mixture was stirred at room temperature for 1 hour, and acetic anhydride (10 mL) and toluene (10 mL) were added thereto. The obtained mixture was stirred under reflux conditions for 6 hours. After the reaction solution was cooled to room temperature, the obtained solid was filtered, and subsequently washed with each of water (30 mL) and acetone (30 mL) to obtain 2.1 g of a compound (1). The chemical structure of the compound (1) is shown below.

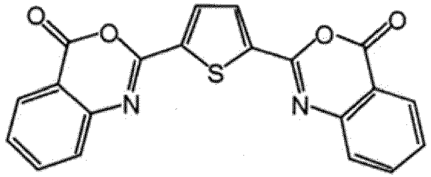

### <Synthesis of compound (2)>

2-amino-5-methylbenzoic acid (1.3 g) was dissolved in dimethylacetamide (12 mL), and 2,5-thiophenedicarbonyl dichloride (1.0 g) was added thereto at room temperature. The obtained mixture was stirred at room temperature for 1 hour, and acetic anhydride (10 mL) and toluene (10 mL) were added thereto. The obtained mixture was stirred under reflux conditions for 6 hours. After the reaction solution was cooled to room temperature, the obtained solid was filtered, and subsequently washed with each of water (20 mL) and acetone (40 mL) to obtain 2.0 g of a compound (2). The chemical structure of the compound (2) is shown below.

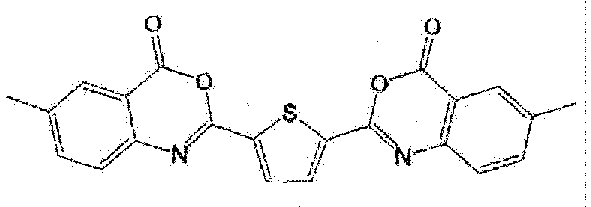

### <Synthesis of compound (3)>

2-aminobenzamide (10 g) was dissolved in dimethylacetamide (50 mL), and 2,5-thiophenedicarbonyl dichloride (7.7 g) was added thereto at room temperature. The obtained mixture was stirred at room temperature for 2 hours, and then an aqueous solution obtained by dissolving cesium carbonate (16 g) in water (60 mL) was added thereto. The obtained mixture was stirred at 70°C for 2 hours. After the reaction solution was cooled to room temperature, water and hydrochloric acid were added thereto. The obtained solid was filtered, and subsequently washed with each of water (30 mL) and acetone (30 mL) to obtain 8.1 g of a compound (3). The chemical structure of the compound (3) is shown below.

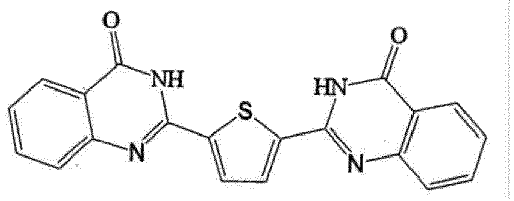

### <Synthesis of compound (4)>

Anthranilic acid (1.3 g) was dissolved in dimethylacetamide (12 mL), and 2,5-furandicarbonyl dichloride (1.0 g) was added thereto at room temperature. The obtained mixture was stirred at room temperature for 1 hour, and acetic anhydride (10 mL) and toluene (10 mL) were added thereto. The obtained mixture was stirred under reflux for 6 hours. After the reaction solution was cooled to room temperature, the obtained solid was filtered, and subsequently washed with each of water (30 mL) and acetone (30 mL) to obtain 2.0 g of a compound (4). The chemical structure of the compound (4) is shown below.

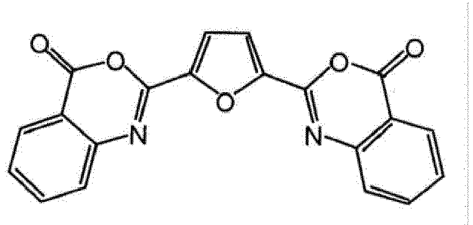

### <Synthesis of compound (5)>

Anthranilic acid (1.6 g) was dissolved in cyclohexanone (25 mL), and sodium carbonate (2.5 g) was added thereto. A solution containing 2,5-thiophenedicarbonyl dichloride (1.2 g) and cyclohexanone (4 mL) was added to the obtained mixture at room temperature. The obtained mixture was stirred at room temperature for 30 minutes and then at 80°C for 1 hour. The obtained solid was filtered and subsequently washed with water (30 mL) to obtain a synthetic intermediate A (2.0 g). The chemical structure of the synthetic intermediate A is shown below.

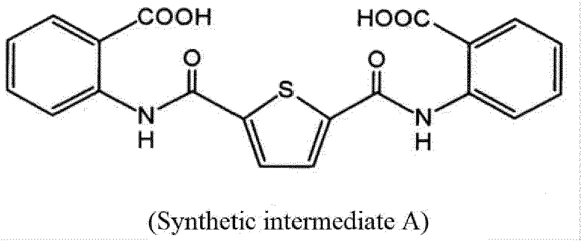

Next, acetic anhydride (10 mL) and toluene (10 mL) were added to the synthetic intermediate A (2.0 g). The obtained mixture was stirred under reflux for 6 hours. After the reaction solution was cooled to room temperature, the obtained solid was filtered, and subsequently washed with each of water (30 mL) and acetone (30 mL) to obtain 1.5 g of a compound (5). The chemical structure of the compound (5) is shown below.

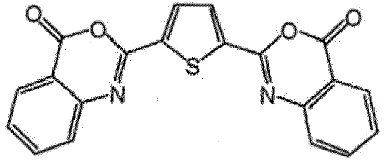

### <Synthesis of compound (6)>

Anthranilic acid (1.3 g) was dissolved in dimethylacetamide (12 mL), and 2,5-thiophenedicarbonyl dichloride (1.0 g) was added thereto at room temperature. The obtained mixture was stirred at room temperature for 1 hour, and acetic anhydride (10 mL) and toluene (10 mL) were added thereto. The obtained mixture was stirred under reflux conditions for 6 hours. After the reaction solution was cooled to room temperature, the obtained solid was filtered, subsequently washed with each of water (30 mL) and acetone (30 mL), and further washed again with each of water (30 mL) and acetone (30 mL) to obtain 2.1 g of a compound (6). The chemical structure of the compound (6) is shown below.

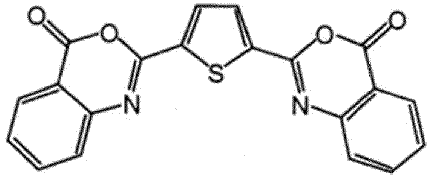

### <Synthesis of compound (7)>

Anthranilic acid (1.3 g) was dissolved in dimethylacetamide (12 mL), and 2,5-thiophenedicarbonyl dichloride (1.0 g) was added thereto at room temperature. The obtained mixture was stirred at room temperature for 1 hour, and acetic anhydride (10 mL) and toluene (10 mL) were added thereto. The obtained mixture was stirred under reflux conditions for 6 hours. After the reaction solution was cooled to room temperature, the obtained solid was filtered, and subsequently washed with each of methanol (30 mL), water (30 mL), and acetone (30 mL) to obtain 2.0 g of a compound (7). The chemical structure of the compound (7) is shown below.

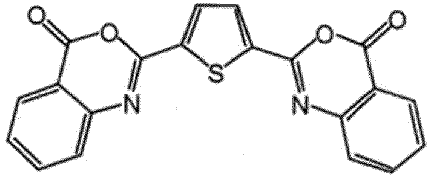

### <Synthesis of Comparative compound (1)>

Anthranilic acid (1.3 g) was dissolved in dimethylacetamide (12 mL), and 2,5-thiophenedicarbonyl dichloride (1.0 g) was added thereto at room temperature. The obtained mixture was stirred at room temperature for 1 hour, and acetic anhydride (10 mL) and toluene (10 mL) were added thereto. The obtained mixture was stirred under reflux conditions for 6 hours. After the reaction solution was cooled to room temperature, the obtained solid was filtered, and subsequently washed with acetone (30 mL) to obtain 2.1 g of a comparative compound (1). The chemical structure of the comparative compound (1) is shown below.

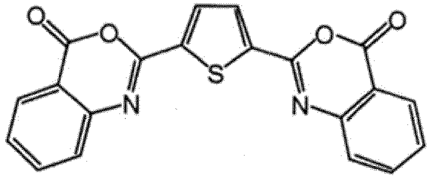

### <Synthesis of Comparative compound (2)>

2-amino-5-methylbenzoic acid (1.3 g) was dissolved in dimethylacetamide (12 mL), and 2,5-thiophenedicarbonyl dichloride (1.0 g) was added thereto at room temperature. The obtained mixture was stirred at room temperature for 1 hour, and acetic anhydride (10 mL) and toluene (10 mL) were added thereto. The obtained mixture was stirred under reflux conditions for 6 hours. After the reaction solution was cooled to room temperature, the obtained solid was filtered, and subsequently washed with acetone (30 mL) to obtain 2.0 g of a comparative compound (2). The chemical structure of the comparative compound (2) is shown below.

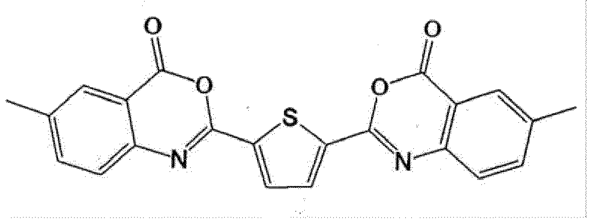

### <Synthesis of comparative compound (3)>

Anthranilic acid (1.6 g) was dissolved in cyclohexanone (25 mL), and sodium carbonate (2.5 g) was added thereto. A solution containing 2,5-thiophenedicarbonyl dichloride (1.2 g) and cyclohexanone (4 mL) was added to the obtained mixture at room temperature. The obtained mixture was stirred at room temperature for 30 minutes and then at 80°C for 1 hour. The obtained solid was filtered and subsequently washed with water (30 mL) to obtain a synthetic intermediate A (2.0 g). The chemical structure of the synthetic intermediate A is shown below.

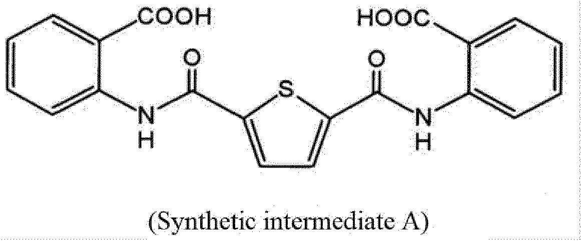

Next, acetic anhydride (10 mL) and toluene (10 mL) were added to the synthetic intermediate A (2.0 g). The obtained mixture was stirred under reflux for 6 hours. After the reaction solution was cooled to room temperature, the obtained solid was filtered, and subsequently washed with acetone (30 mL) to obtain 1.5 g of a comparative compound (3). The chemical structure of the comparative compound (3) is shown below.

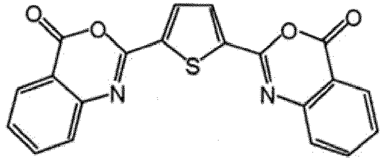

### <Measurement of content of ion in compound>

The content of ions in each of the above-described compounds was measured according to the following method. The measurement results are shown in Table 1.

### [Chloride ion]

Using a combustion device "AQF-100" manufactured by Mitsubishi Chemical Corporation and an ion chromatography "ICS-1500" manufactured by Dionex Corporation, the content of chloride ions was quantified by combustion ion chromatography.

### [Metal ion]

Sodium ions, aluminum ions, iron ions, and calcium ions were quantified by a frameless atomic absorption method using a polarized Zeeman atomic absorption spectrophotometer "ZA3700" manufactured by Hitachi, Ltd.

**[Table 1]**

| Sample number | Compound | Content of ion (ppm, in terms of mass) | | | | |
|---|---|---|---|---|---|---|
| | | Cl | Na | Al | Fe | Ca |
| 1 | Compound (1) | 13.5 | 0.3 | 0.1 | 0.4 | 0.3 |
| 2 | Compound (2) | 8.7 | 0.5 | 0.1 | 0.7 | 0.5 |
| 3 | Compound (3) | 17.3 | 0.7 | 0.2 | 0.3 | 0.6 |
| 4 | Compound (4) | 9.2 | 0.4 | 0.1 | 0.3 | 0.3 |
| 5 | Compound (5) | 20.8 | 15.3 | 0.2 | 0.5 | 0.4 |
| 6 | Compound (6) | 11.1 | 0.2 | 0.1 | 0.3 | 0.3 |
| 7 | Compound (7) | 9.5 | 0.2 | 0.1 | 0.3 | 0.3 |
| 8 | Comparative compound (1) | 73.2 | 33.7 | 2.1 | 2.2 | 4.3 |
| 9 | Comparative compound (2) | 66.7 | 45.9 | 3.5 | 2.5 | 7.6 |
| 10 | Comparative compound (3) | 95.6 | 275.3 | 2.8 | 3.1 | 5.1 |

### <Production of polyethylene terephthalate film>

### [Example 1]

A pellet (90 parts by mass) of polyethylene terephthalate (PET, weight-average molecular weight: 30,000) dried at 160°C for 8 hours was mixed with the dried compounds (1) (10 parts by mass), and the resultant mixture was charged into an extruder. The mixture was subjected to melt kneading at 280°C to prepare a pellet containing polyethylene terephthalate and the compound (1) (hereinafter, referred to as a "pellet (A)" in this paragraph). Next, a PET pellet dried at 160°C for 8 hours was mixed with the pellet (A). The content of the compound (1) was 1 part by mass with respect to the total mass (100 parts by mass) of the mixture of the PET pellet and the pellet (A). The mixture was subjected to melt kneading at 280°C to produce a film (one form of the composition in the present disclosure) having a thickness of 50 µm according to a melt extrusion method.

### [Example 2]

A film was produced according to the same procedure as in Example 1 except that the compound (2) was used instead of the compound (1).

### [Example 3]

A film was produced according to the same procedure as in Example 1 except that the compound (3) was used instead of the compound (1).

### [Example 4]

A film was produced according to the same procedure as in Example 1 except that the compound (4) was used instead of the compound (1).

### [Example 5]

A film was produced according to the same procedure as in Example 1 except that the compound (5) was used instead of the compound (1).

### [Example 6]

A film was produced according to the same procedure as in Example 1 except that the compound (6) was used instead of the compound (1).

### [Example 7]

A film was produced according to the same procedure as in Example 1 except that the compound (7) was used instead of the compound (1).

### [Comparative Example 1]

A film was produced according to the same procedure as in Example 1 except that the comparative compound (1) was used instead of the compound (1).

### [Comparative Example 2]

A film was produced according to the same procedure as in Example 1 except that the comparative compound (2) was used instead of the compound (1).

### [Comparative Example 3]

A film was produced according to the same procedure as in Example 1 except that the comparative compound (3) was used instead of the compound (1).

### <Evaluation of polyethylene terephthalate film>

The following evaluations were carried out using the polyethylene terephthalate films produced in Examples and Comparative Examples. The evaluation results are shown in Table 2.

### [Ion content]

Chloride ions and sodium ions in each film were quantified according to the method described above.

### [Tint (a*)]

Using a spectrophotometer "UV-2500PC" manufactured by Shimadzu Corporation, the value of the a* of each film was measured, and the tint was evaluated according to the following criteria.
A: Less than -2.0
B: -2.0 or more and less than 0
C: 0 or more and less than 2.0
D: 2.0 or more

### [Haze]

Using a haze meter "NDH 7000" manufactured by NIPPON DENSHOKU INDUSTRIES Co., Ltd., the haze of each film was measured, and the haze was evaluated according to the following criteria.
A: 0.0% or more and less than 0.5%
B: 0.5% or more and less than 1.0%
C: 1.0% or more and less than 5.0%
D: 5.0% or more

### [Change in intrinsic viscosity]

A test piece of each film was subjected to heat treatment at 280°C for 60 minutes in a nitrogen atmosphere, and then the intrinsic viscosity of each test piece (hereinafter, referred to as "the intrinsic viscosity of the film after the heat treatment") was measured. The intrinsic viscosity was measured using an Ostwald viscometer using o-chlorophenol as a solvent under the condition of 25°C. The difference (ΔIV) between the intrinsic viscosity of the film after the heat treatment and the intrinsic viscosity of the film before the heat treatment was determined. It is meant that the larger the value of ΔIV is, the more the physical properties of the polymer compound are changed.

**[Table 2]**

| | Kind of compound | Content of ion [ppm, in terms of mass] | | Appearance | | Change in intrinsic viscosity (ΔIV) |
|---|---|---|---|---|---|---|
| | | Cl | Na | Tint (a*) | Haze | |
| Example 1 | Compound (1) | 1.1 | 0.9 | A | A | 0.09 |
| Example 2 | Compound (2) | 0.9 | 1.0 | A | A | 0.10 |
| Example 3 | Compound (3) | 1.2 | 1.0 | A | A | 0.08 |
| Example 4 | Compound (4) | 0.9 | 0.9 | A | A | 0.09 |
| Example 5 | Compound (5) | 1.2 | 1.2 | B | B | 0.07 |
| Example 6 | Compound (6) | 0.9 | 0.9 | A | A | 0.08 |
| Example 7 | Compound (7) | 0.9 | 0.9 | A | A | 0.08 |
| Comparative Example 1 | Comparative compound (1) | 1.7 | 1.5 | C | C | 0.25 |
| Comparative Example 2 | Comparative compound (2) | 1.7 | 1.6 | D | C | 0.23 |
| Comparative Example 3 | Comparative compound (3) | 2.0 | 3.8 | D | D | 0.30 |

The results shown in Table 2 indicate that the redness is small in Examples 1 to 7 as compared with Comparative Examples 1 to 3.

### <Production of polycarbonate plate>

### [Example 8]

A pellet (90 parts by mass) of polycarbonate (PC, weight-average molecular weight: 30,000) was mixed with the dried compound (1) (10 parts by mass), and the resultant mixture was charged into an extruder. The mixture was subjected to melt kneading at 290°C to prepare a pellet containing polycarbonate and the compound (1) (hereinafter, referred to as a "pellet (B)" in this paragraph). Next, the PC pellet was mixed with the pellet (B). The content of the compound (1) was 1 part by mass with respect to the total mass (100 parts by mass) of the mixture of the PC pellet and the pellet (B). An injection molding machine was used to carry out injection molding at a cylinder temperature of 290°C to produce a plate (one form of the composition in the present disclosure) having a thickness of 1 mm.

### [Example 9]

A plate was produced according to the same procedure as in Example 8 except that the compound (2) was used instead of the compound (1).

### [Example 10]

A plate was produced according to the same procedure as in Example 8 except that the compound (3) was used instead of the compound (1).

### [Example 11]

A plate was produced according to the same procedure as in Example 8 except that the compound (4) was used instead of the compound (1).

### [Example 12]

A plate was produced according to the same procedure as in Example 8 except that the compound (5) was used instead of the compound (1).

### [Example 13]

A plate was produced according to the same procedure as in Example 8 except that the compound (6) was used instead of the compound (1).

### [Example 14]

A plate was produced according to the same procedure as in Example 8 except that the compound (7) was used instead of the compound (1).

### [Comparative Example 4]

A plate was produced according to the same procedure as in Example 8 except that the comparative compound (1) was used instead of the compound (1).

### [Comparative Example 5]

A plate was produced according to the same procedure as in Example 8 except that the comparative compound (2) was used instead of the compound (1).

### [Comparative Example 6]

A plate was produced according to the same procedure as in Example 8 except that the comparative compound (3) was used instead of the compound (1).

### <Evaluation of polycarbonate plate>

The following evaluations were carried out using the polycarbonate plates prepared in the above-described Examples and Comparative Examples. The evaluation results are shown in Table 3.

### [Ion content]

Chloride ions and sodium ions in each plate were quantified according to the method described above.

### [Tint (a*)]

Using a spectrophotometer "UV-2500PC" manufactured by Shimadzu Corporation, the value of the a* of each plate was measured, and the tint was evaluated according to the following criteria.
A: Less than -2.0
B: -2.0 or more and less than 0
C: 0 or more and less than 2.0
D: 2.0 or more

### [Haze]

Using a haze meter "NDH 7000" manufactured by NIPPON DENSHOKU INDUSTRIES Co., Ltd., the haze of each plate was measured, and the haze was evaluated according to the following criteria.
A: 0.0% or more and less than 0.5%
B: 0.5% or more and less than 1.0%
C: 1.0% or more and less than 5.0%
D: 5.0% or more

**[Table 3]**

| | Kind of compound | Content of ion [ppm, in terms of mass] | | Appearance | |
|---|---|---|---|---|---|
| | | Cl | Na | Tint (a*) | Haze |
| Example 8 | Compound (1) | 1.0 | 1.0 | A | A |
| Example 9 | Compound (2) | 1.0 | 0.9 | A | A |
| Example 10 | Compound (3) | 1.1 | 1.1 | A | B |
| Example 11 | Compound (4) | 0.9 | 1.0 | A | A |
| Example 12 | Compound (5) | 1.3 | 1.2 | B | B |
| Example 13 | Compound (6) | 0.9 | 0.9 | A | A |
| Example 14 | Compound (7) | 0.9 | 0.9 | A | A |
| Comparative Example 4 | Comparative compound (1) | 1.8 | 1.6 | C | C |
| Comparative Example 5 | Comparative compound (2) | 1.7 | 1.7 | C | C |
| Comparative Example 6 | Comparative compound (3) | 2.1 | 4.0 | D | D |

The results shown in Table 3 indicate that the redness is small in Examples 8 to 14 as compared with Comparative Examples 4 to 6.

The disclosure of JP2020-101102 filed on June 10, 2020 is incorporated in the present specification by reference in its entirety. All documents, patent applications, and technical standards described in the present specification are herein incorporated by reference to the same extent that individual documents, patent applications, and technical standards have been specifically and individually indicated to be incorporated by reference, respectively.

## Claims

1. A composition comprising:
a compound represented by General Formula (1); and
a polymer compound,
wherein a content of a chloride ion is less than 1.5 ppm with respect to a total mass of the composition, 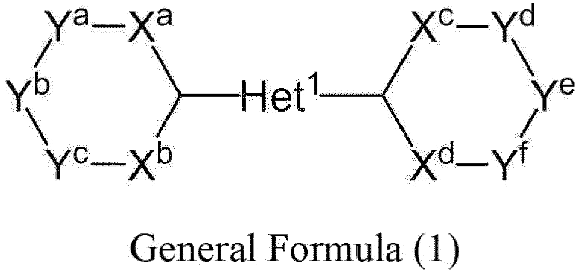
in General Formula (1), Het¹ represents a divalent aromatic heterocyclic residue of a 5-membered ring or a 6-membered ring, X^{a}, X^{b}, X^{c}, and X^{d} each independently represent a heteroatom, Y^{a}, Y^{b}, Y^{c}, Y^{d}, Y^{e}, and Y^{f} each independently represent a heteroatom or a carbon atom, and two 6-membered rings bonded to Het¹ each independently may have a double bond.

2. The composition according to claim 1,
wherein a content of a sodium ion is less than 1.5 ppm with respect to the total mass of the composition.

3. The composition according to claim 1 or 2,
wherein the compound represented by General Formula (1) is a compound represented by General Formula (2), 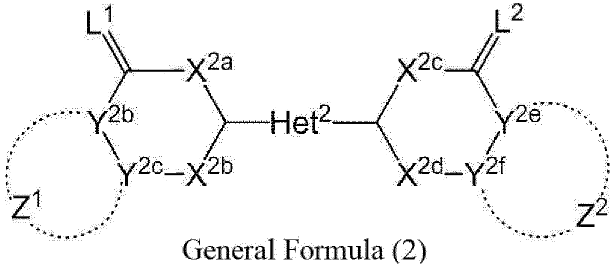
in General Formula (2), Het² represents a divalent aromatic heterocyclic residue of a 5-membered ring or a 6-membered ring, X^{2a}, X^{2b}, X^{2c}, and X^{2d} each independently represent a heteroatom, Y^{2b}, Y^{2c}, Y^{2e}, and Y^{2f} each independently represent a heteroatom or a carbon atom, two 6-membered rings bonded to Het² may each independently have a double bond, L¹ and L² each independently represent an oxygen atom, a sulfur atom, or NR^{a}, where R^{a} represents a hydrogen atom or a monovalent substituent, Z¹ represents an atomic group that is required for bonding to Y^{2b} and Y^{2c} to form a 4-membered ring to an 8-membered ring, and Z² represents an atomic group that is required for bonding to Y^{2e} and Y^{2f} to form a 4-membered ring to an 8-membered ring.

4. The composition according to claim 3,
wherein the compound represented by General Formula (2) is a compound represented by General Formula (3), 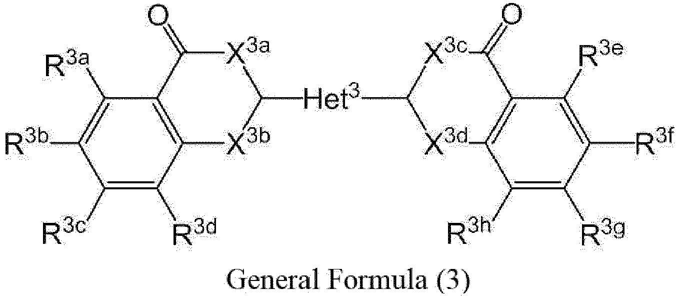
in General Formula (3), Het³ represents a divalent aromatic heterocyclic residue of a 5-membered ring or a 6-membered ring, X^{3a}, X^{3b}, X^{3c}, and X^{3d} each independently represent a heteroatom, X^{3a} or X^{3b} may form a double bond with a carbon atom in a ring bonded to Het³, X^{3c} or X^{3d} may form a double bond with a carbon atom in a ring bonded to Het³, and R^{3a}, R^{3b}, R^{3c}, R^{3d}, R^{3e}, R^{3f}, R^{3g}, and R^{3h} each independently represent a hydrogen atom or a monovalent substituent.

5. The composition according to claim 4,
wherein the compound represented by General Formula (3) is a compound represented by General Formula (4), 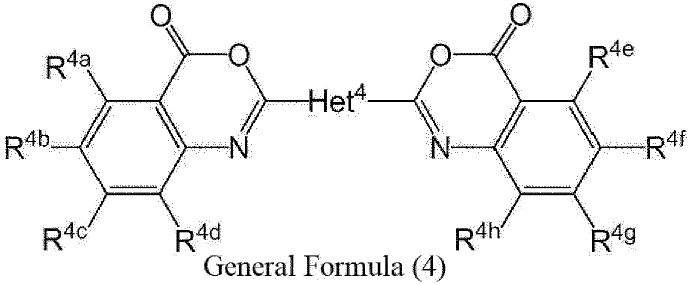
in General Formula (4), Het⁴ represents a divalent aromatic heterocyclic residue of a 5-membered ring or a 6-membered ring, and R^{4a}, R^{4b}, R^{4c}, R^{4d}, R^{4e}, R^{4f}, R^{4g}, and R^{4h} each independently represent a hydrogen atom or a monovalent substituent.

6. The composition according to claim 5,
wherein the compound represented by General Formula (4) is a compound represented by General Formula (5), 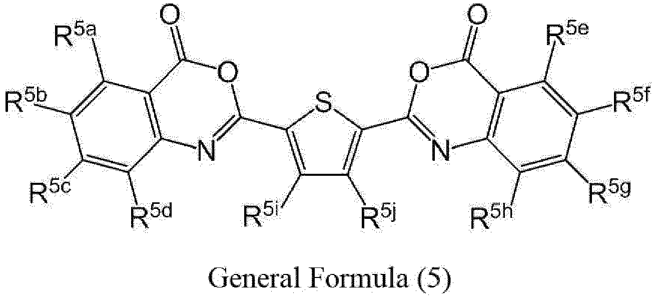
in General Formula (5), R^{5a}, R^{5b}, R^{5c}, R^{5d}, R^{5e}, R^{5f}, R^{5g} and R^{5h} each independently represent a hydrogen atom or a monovalent substituent, and R⁵ⁱ and R^{5j} each independently represent a hydrogen atom or a monovalent substituent.

7. The composition according to any one of claims 1 to 6,
wherein the polymer compound is at least one selected from the group consisting of polyester, polycarbonate, polyethylene, polypropylene, an acrylic resin, cyclic polyolefin, an epoxy resin, polyurethane, polythiourethane, polyimide, polyamide, and a fluororesin.

8. The composition according to any one of claims 1 to 7,
wherein the composition is for a lens for spectacles.

9. The composition according to any one of claims 1 to 7,
wherein the composition is for an optical lens.

10. The composition according to any one of claims 1 to 7,
wherein the composition is for an optical film.

11. A compound represented by General Formula (1), General Formula (2), General Formula (3), General Formula (4), or General Formula (5),
wherein a content of a chloride ion is less than 30 ppm in terms of mass, 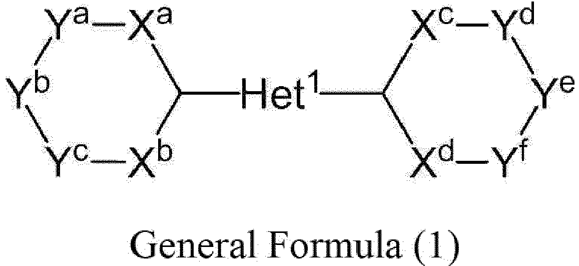
in General Formula (1), Het¹ represents a divalent aromatic heterocyclic residue of a 5-membered ring or a 6-membered ring, X^{a}, X^{b}, X^{c}, and X^{d} each independently represent a heteroatom, Y^{a}, Y^{b}, Y^{c}, Y^{d}, Y^{e}, and Y^{f} each independently represent a heteroatom or a carbon atom, and two 6-membered rings bonded to Het¹ each independently may have a double bond. 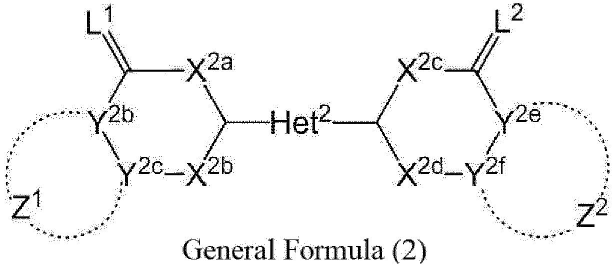
in General Formula (2), Het² represents a divalent aromatic heterocyclic residue of a 5-membered ring or a 6-membered ring, X^{2a}, X^{2b}, X^{2c}, and X^{2d} each independently represent a heteroatom, Y^{2b}, Y^{2c}, Y^{2e}, and Y^{2f} each independently represent a heteroatom or a carbon atom, two 6-membered rings bonded to Het² may each independently have a double bond, L¹ and L² each independently represent an oxygen atom, a sulfur atom, or NR^{a}, where R^{a} represents a hydrogen atom or a monovalent substituent, Z¹ represents an atomic group that is required for bonding to Y^{2b} and Y^{2c} to form a 4-membered ring to an 8-membered ring, and Z² represents an atomic group that is required for bonding to Y^{2e} and Y^{2f} to form a 4-membered ring to an 8-membered ring, 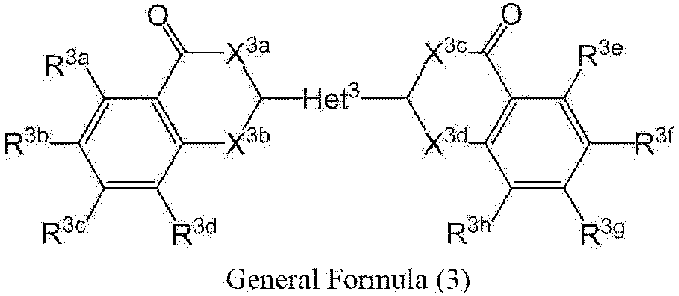
in General Formula (3), Het³ represents a divalent aromatic heterocyclic residue of a 5-membered ring or a 6-membered ring, X^{3a}, X^{3b}, X^{3c}, and X^{3d} each independently represent a heteroatom, X^{3a} or X^{3b} may form a double bond with a carbon atom in a ring bonded to Het³, X^{3c} or X^{3d} may form a double bond with a carbon atom in a ring bonded to Het³, and R^{3a}, R^{3b}, R^{3c}, R^{3d}, R^{3e}, R^{3f}, R^{3g}, and R^{3h} each independently represent a hydrogen atom or a monovalent substituent, 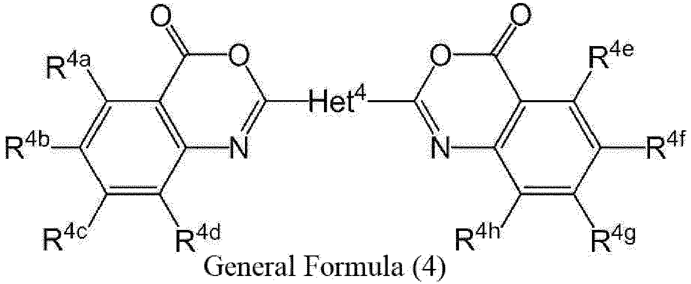
in General Formula (4), Het⁴ represents a divalent aromatic heterocyclic residue of a 5-membered ring or a 6-membered ring, and R^{4a}, R^{4b}, R^{4c}, R^{4d}, R^{4e}, R^{4f}, R^{4g}, and R^{4h} each independently represent a hydrogen atom or a monovalent substituent, 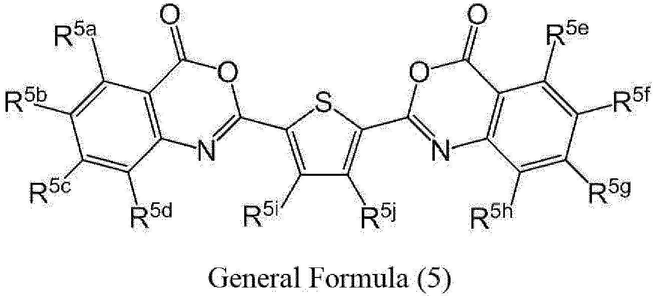
in General Formula (5), R^{5a}, R^{5b}, R^{5c}, R^{5d}, R^{5e}, R^{5f}, R^{5g} and R^{5h} each independently represent a hydrogen atom or a monovalent substituent, and R⁵ⁱ and R^{5j} each independently represent a hydrogen atom or a monovalent substituent.

12. The compound according to claim 11,
wherein a content of a sodium ion is less than 1 ppm in terms of mass, a content of an aluminum ion is less than 0.5 ppm in terms of mass, a content of an iron ion is less than 0.5 ppm in terms of mass, and a content of a calcium ion is less than 1 ppm in terms of mass.
